# EUROPEAN PATENT APPLICATION

(11) **EP 4 796 180 A1**
(43) Date of publication of application: **26.08.2026**
(21) Application number: 25159392.7
(22) Date of filing: 21.02.2025
(51) Int. Cl.: A61P 31/22, C07K 16/08

(54) **A THERAPEUTIC VHH ANTIBODY THAT BLOCKS INFECTION BY HERPES SIMPLEX VIRUSES 1 AND 2**

(71) Applicant: Max-Planck-Gesellschaft zur Förderung der Wissenschaften e.V., 80539 München (DE); Leibniz-Institut für Virologie, 20251 Hamburg (DE)
(72) Inventor: The designation of the inventor has not yet been filed
(74) Representative: Weickmann & Weickmann PartmbB

(57) **Abstract**

The present invention pertains to the fields of antibody technology, medicine, pharmacology, infection biology, and medical diagnostics. More specifically, the present disclosure provides a VHH antibody that neutralizes herpes simplex viruses HSV-1 and HSV-2 by blocking the pre-fusion state of the gB fusion protein, as well as methods for using this VHH against HSV infections.

## Description

### Field of the invention

The present invention pertains to the fields of antibody technology, medicine, pharmacology, infection biology, and medical diagnostics. More specifically, the present disclosure provides a VHH antibody that neutralizes herpes simplex viruses HSV-1 and HSV-2 by binding and arresting the pre-fusion form of their viral fusion protein gB, which in turn blocks viral entry into a host cell.

### Background of the invention

Herpesviruses are a severe burden to the global public health. Nine different herpesviruses infect humans, including important human pathogens like herpes simplex virus 1 and 2 (HSV-1, HSV-2), Varicella Zoster Virus (VZV), Human Cytomegalovirus (HCMV) and Epstein-Barr-virus (EBV). One of the hallmarks of herpesvirus infections is latency, meaning that the virus stays in the body in a latent state for life, concealed from the host's immune system. Although latency is asymptomatic, the virus can be reactivated at any time by often poorly characterised stimuli.

In 2016, an estimated 3.6 billion people were infected with HSV-1 (prevalence of 64% worldwide), while an estimated 17% had genital HSV-1 or -2 infections, disproportionally affecting women (James *et al,* 2020). In the United States alone, an estimated 600 000 people become infected with HSV-2 every year. In addition to higher transmission rates of HIV in HSV-2 infected people, these viruses can cause significant morbidity and mortality in immunocompromised patients (with AIDS, cancer or autoimmune disease).

Typically, initial infections with HSV-1 or HSV-2 occur through close contacts at the oral or genital mucosa. Here, the virus replicates in epithelial cells until, after a 4-6 days incubation time, lysis of the infected cells and inflammation begin (Whitley, 1996). Viral particles will then progress to infect sensory neuronal cells, where they establish latency by travelling to the cell body to deposit the viral genome as an extrachromosomal element in the nucleus. Due to the fact that these neurons do not divide and there is no active production of viral particles, the virus can stay dormant, undetected by the immune system. From there, the virus can be reactivated and travel along the axons of the neuron back to the initial site of primary infection to infect and replicate in epithelial cells (Whitley, 1996).

Mucocutaneous infections are the most common clinical manifestations caused by HSV-1 and HSV-2. Recurring reactivation of HSV-1 can lead to oropharyngeal infections and cold sores, while HSV-2 is usually the cause of genital herpes, although cases attributed to HSV-1 are rising (Whitley, 1996). Herpes infections are permanent, with often painful recurrences, associated with social stigma; they significantly impair the quality of life of those affected. Its tropism allows HSV also to infect the eye, causing conjunctivitis and herpetic keratitis, which is the most common infectious cause of blindness in the United States (Whitley, 1996).

Because of their high prevalence, herpesviruses also pose a constant threat for patients receiving organ transplants. Due to the ageing population and increased application of immunosuppressive therapies, the number of individuals susceptible to these infections will rise.

In rare cases (1 in 150,000 individuals) of primary infection or re-activation, HSV invades the central nervous system and causes herpes simplex encephalitis, (Whitley, 1996; Skouboe *et al*, 2023). HSV-1 is responsible for 90% of these cases and the most common cause of viral encephalitis, accounting for 10-20% of cases (Levitz, 1998). Although the introduction of acyclovir has reduced the mortality rate, it remains high and survivors often suffer temporary or permanent neurological sequelae, including significant cognitive impairments (Whitley *et al*, 1991).

In addition, HSV-2 is a leading cause of aseptic meningitis, which often occurs during primary infection (Bodilsen *et al*, 2018). These primary infections, but also recurring activations of genital herpes, pose a risk of transmission during childbirth causing neonatal herpes, which happens in ~1/3000 deliveries each year in the United States. The majority of infections are attributed to HSV-2 transmitted through contact of the newborn with genital secretions during birth. The infection manifests as either skin, eye and mouth disease; encephalitis or disseminated infection. Skin, eye, and mouth disease typically represent superficial lesions and do not affect other organ systems, while both encephalitis and disseminated infection can involve the central nervous system. Disseminated infection affects multiple organ systems and can lead to severe complications including intravascular coagulation, haemorrhagic pneumonitis, cutaneous lesions, and encephalitis, which typically manifests as a diffuse condition (Whitley, 1996). Mortality varies widely among these categories: skin, eye, and mouth disease have a mortality rate close to zero, while encephalitis carries a 15% mortality rate, and disseminated infection has a mortality rate of up to 60% in neonates. Furthermore, the morbidity associated with these infections is noteworthy; even with appropriate antiviral therapy, only about 40% of children affected by encephalitis or disseminated disease develop normally (Whitley, 1996).

Several anti-herpesviral agents have been approved and are currently in use, namely acyclovir, ganciclovir, penciclovir, cidofovir and foscarnet. All these drugs inhibit the viral DNA polymerase, i.e. they target viral DNA-replication during lytic infection. They are only indicated against HSV, VZV and HCMV. The increased use of these drugs is leading to the emergence of resistant viral strains (Gohring *et al*, 2006), particularly in immunocompromised hosts. Furthermore, these drugs are only effective after viral infection and cannot be used as a prophylactic measure.

The high prevalence of herpesvirus infections and the limited number of effective antivirals highlight the urgent need for the development of new anti-herpes viral agents targeting alternative steps in the herpesvirus life cycle.

The first step in HSV infection is the attachment of virus particles to heparan sulphate and some forms of dermatan sulphate on the surface of the host cell. This initial interaction is mediated by the viral glycoproteins C (gC) and B (gB) (Herold *et al*, 1994).

Although gC increases the efficiency during this first step, it is not essential (Herold *et al*, 1991). Once a stable connection has been established, the virus has to fuse its own membrane with the plasma membrane of the host cell. In contrast to many enveloped viruses like Influenza or SARS-CoV-2 that use a single protein for attachment and fusion, herpesviruses distribute the different requirements of the entry process to different proteins. In HSV, these are the glycoprotein D (gD), as well as a complex consisting of glycoprotein H and L (gH/L) and gB (Davis-Poynter *et al*, 1994).

gD strengthens the interaction on the surface by direct interaction with the cell surface receptors HVEM or Nectin-1 (Krummenacher *et al*, 2004). Potentially triggered by gD, gH/L is believed to transmit an activating signal to gB that initiates the fusion process (Heldwein, 2016). Although the structural conservation of gH/L is low, it is still found in all herpesviruses, while gD is only found in alpha herpesviruses (e.g. HSV-1, HSV-2 or VZV).

The *bona fide* membrane fusion protein of these four glycoproteins is gB, making it the only essential and structurally highly conserved glycoprotein on the membrane of all herpesviruses. This homotrimeric type I transmembrane protein is essential for infection as it catalyses the fusion process between the viral and host cell membrane (Rey, 2006). Fusion of the two membranes is a prerequisite for the release of the viral genome-containing capsid into the host cell.

gB is initially present on the viral surface in a metastable, high-energy pre-fusion form. Activation, probably mediated by gH/L, causes a substantial rearrangement, which exposes hydrophobic 'fusion loops' that are inserted into the target membrane. The protein then undergoes further conformational changes to the energetically favoured hairpin or post-fusion conformation, providing energy to pull the membranes together, leading to fusion and release of the viral contents. Thus, the protein usually adapts one of two conformations - pre- or post-fusion. Once the conformational change is triggered, the protein is trapped in the low-energy post-fusion conformation, unable to perform its function again. On the surface of the infectious virus, gB is found in its pre-fusion form, which positions this structure as a key target for the immune response. Because of its critical function in the infection process and significant structural conservation, gB stands out as an excellent target for antiviral intervention.

During the long co-evolution of herpesviruses with humans, these viruses have developed ways to evade detection and neutralization by the immune system. This includes that gB is hard to target with neutralizing antibodies, because critical regions are inaccessible and/or protected by glycosylation. Yet, targeting epitopes on the pre-fusion structure has a high potential of a neutralizing effect by blocking the necessary domain rearrangements. The transition from pre- to post-fusion causes a dramatic change in the protein's overall shape, while the individual domains retain their structure. This means that potential pre-fusion-specific epitopes have to be located either in linking regions that connect the rigid domains or distributed across multiple domains that are close together in the pre-fusion state, but far apart in the post-fusion conformation. The fact that no pre-fusion-specific antibodies have so far been described for any herpesvirus is illustrating how difficult a targeting of such epitopes is.

Nanobodies (VHH antibodies) correspond to the antigen-binding domains of camelid heavy chain-only antibodies (Hamers-Casterman *et al*, 1993; Muyldermans, 2013). They feature several key advantages over traditional antibodies. First, their size is just ¼ (~13 kDa) of that of a Fab fragment. This allows them to target epitopes in cavities that are too narrow to be reached by normal antibodies. This is of particular relevance for the present invention. Second, the single-domain structure of VHH antibodies does not require subunit assembly and confers more robust folding, allowing a production also in bacteria or yeast. Certain VHH antibodies proved to be hyper-thermostable and display a particularly high affinity (Guttler *et al*, 2021).

### Summary of the invention

A first aspect of the present invention provides an VHH antibody specifically recognizing a pre-fusion state of a herpesvirus polypeptide gB. In certain embodiments, the VHH antibody is directed against a pre-fusion state of the HSV-1 and HSV-2 polypeptide gB.

A further aspect of the invention relates to a VHH antibody that neutralizes HSV-1 and HSV-2. The neutralizing activity is based on the specific recognition of a structural epitope that is only available on the surface of the gB protein in the pre-fusion state.

By this, only the conformation able to perform the fusion function (pre-fusion state) is targeted. A transition to the post-fusion form might be prevented by steric clashes of the VHH with the target in post-fusion conformation or at least by imposing an energetic barrier for such a transition.

In certain embodiments, the VHH antibody is in monovalent form, e.g., as a single domain VHH antibody. The use in a monovalent format is possible due to the very high affinity of the antibody. The antibody may be fused to a heterologous moiety, e.g., a heterologous protein such as an immunoglobulin Fc fragment. In certain embodiments, the VHH antibody may be in multivalent form, to possibly enhance beneficial effects of the fused moieties.

In particular embodiments, the VHH antibody is fused to an immunoglobulin Fc fragment and is in a dimeric form.

Preferably, the VHH antibody comprises
(a) a combination of CDR1, CDR2 and CDR3 sequences as in shown in SEQ. ID NO: 14-16 or
(b) a combination of CDR1, CDR2 and CDR3 sequences which has an identity of at least 80%, at least 90% or at least 95% to a combination of CDR1, CDR2 and CDR3 sequences of (a).

Even more preferably, the VHH antibody comprises a VHH sequence as in shown in SEQ. ID NO: 13.

In certain embodiments, the VHH antibody is hyperthermostable.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

In certain embodiments, the non-human organism is a probiotic microorganism, which secretes a VHH antibody as described above and is capable of inhabiting a mammalian, e.g., human mucosa for example the female genital tract.

A further aspect of the present invention provides a VHH antibody, a nucleic acid molecule or a cell as described above for use in medicine, e.g., for use in therapy or diagnostics.

In particular embodiments, the VHH antibody, the nucleic acid or the cell or organism is provided for use in the prevention, treatment and/or mitigation of a condition caused by, associated with and/or accompanied by an infection with HSV-1 or HSV-2.

A further aspect of the present invention provides a method for the prevention, treatment and/or mitigation of a condition caused by, associated with and/or accompanied by an infection with HSV-1 or HSV-2 comprising administering an effective dose of the VHH antibody, the nucleic acid or the cell or organism as described above to a subject in need thereof, particularly to a human subject.

Still a further aspect of the present invention is a probiotic microorganism, which secretes a VHH antibody as described above and is capable of inhabiting a mammalian, e.g., human mucosa, for example the female genital tract.

Still a further aspect of the present invention provides a method for limiting a mucosal HSV infection and HSV transmission by a probiotic microorganism, comprising administering a probiotic microorganism, which secretes a VHH antibody as described above and is capable of inhabiting human mucosae, to a subject being infected with HSV-1 and/or HSV-2 or being at risk of an infection with HSV-1 and/or HSV-2.

Still a further aspect of the present invention is a non-infectious, VHH-fusion-blocked HSV-1 or HSV-2 viral particle, particularly for use as an immunogen for generating a VHH antibody specifically recognizing a pre-fusion state of a herpesvirus polypeptide gB.

### Embodiments of the Invention

In the following, specific embodiments of the invention are disclosed as follows:
1. A VHH antibody which specifically recognizes the pre-fusion state of an HSV-1 gB polypeptide, particularly of an HSV-1 gB polypeptide according to amino acids 31-904 of SEQ ID NO: 1.
2. The VHH antibody of embodiment 1 which further specifically recognises the pre-fusion state of an HSV-2 gB polypeptide, particularly of an HSV-2 gB polypeptide according to amino acids 22-904 of SEQ ID NO: 2.
3. The VHH antibody of embodiment 1 or 2 that neutralizes HSV-1
4. The VHH antibody of embodiment 3 that neutralizes HSV-1 and HSV-2.
5. The VHH antibody of any one of embodiments 1-4 which specifically recognizes an epitope of an HSV-1 gB polypeptide in the pre-fusion state comprising or consisting of amino acids A106, N108, G121, A122, D285, E286, G292, F294, Y296, H311, T312, S313, Y314, A315, D317, P348, K349, N585, Y640, E651, A654, Y655, S656, H657, Q658, L659, and D663 as shown in SEQ ID NO: 1 wherein D285 may be replaced by N285 and/or S312 may be replaced by T313.
6. The VHH antibody of any one of embodiments 1-5 which specifically recognizes an epitope of an HSV-2 gB polypeptide in the pre-fusion state comprising or consisting of amino acids V101, G116, A117, D280, E281, G287, F289, Y291, H306, T307, S308, Y309, A310, R313, P343, N582, Y637, E648, A651, Y652, S653, H654, Q655, and D660 as shown in SEQ ID NO: 2 wherein S308 may be replaced by T308.
7. The VHH antibody of any one of embodiments 1-6 which competes with the VHH antibody Nb1_gbHSV as shown in SEQ ID NO: 13 for binding to an HSV-1 gB polypeptide in the pre-fusion state.
8. The VHH antibody of any one of embodiments 1-7 which competes with the VHH antibody Nb1_gbHSV as shown in SEQ ID NO: 13 for binding to an HSV-2 gB polypeptide in the pre-fusion state.
9. The VHH antibody of any one of embodiments 1-8 which does not bind to the post-fusion state of an HSV-1 gB polypeptide according to amino acids 31-904 of SEQ ID NO: 1 and/or to the post-fusion state of an HSV-2 gB polypeptide according to amino acids 22-904 of SEQ ID NO: 2.
10. The VHH antibody of any one of embodiments 1-9 which comprises
   (a) a combination of CDR1, CDR2 and CDR3 sequences as in shown in SEQ. ID NO: 14-16 or
   (b) a combination of CDR1, CDR2 and CDR3 sequences which has an identity of at least 80%, at least 90% or at least 95% to a combination of CDR1, CDR2 and CDR3 sequences of (a).
11. The VHH antibody of embodiment 10 which comprises a combination of CDR1, CDR2 and CDR3 sequences as in shown in SEQ. ID NO: 14-16.
12. The VHH antibody of any one of embodiments 1-11 which comprises
   (a) a VHH sequence as shown in SEQ. ID NO: 13 or
   (b) a VHH sequence which has an identity of at least 90%, at least 95% or at least 98% to a VHH sequence of (a).
13.The VHH antibody of any one of embodiments 1-12 which comprises a VHH sequence as shown in SEQ. ID NO: 13 or a humanized variant thereof.
14.The VHH antibody of any one of embodiments 10-13 which comprises a VHH sequence comprising amino acids Q1, A26, S27, G28, R29, M32, G34, A35, Y39, L49, T52, T54, D55, R56, S58, T59, N60, Y61, K66, R100, W101, R102, G103, M104 of SEQ ID NO. 13.
15. The VHH antibody of any one of the preceding embodiments which is hyperthermostable and has a melting temperature of at least 95°C.
16. The VHH antibody of any one of the preceding embodiments which is a fully disulfide-bonded VHH antibody.
17. The VHH antibody of any one of the preceding embodiments which is covalently or non-covalently conjugated to a heterologous moiety.
18. The VHH antibody of any one of the preceding embodiments which is fused to an immunoglobulin Fc fragment.
19. The VHH antibody of any one of the preceding embodiments, which is non-glycosylated, or which is glycosylated.
20. The VHH antibody of any one of the preceding embodiments, which is produced in a bacterium, e.g., *E. coli*, or in a yeast, e.g., *Pichia pastoris*, or in a human or animal cell, e.g., an insect cell or a mammalian cell.
21. The VHH antibody of any one of the preceding embodiments, which is in a monovalent format.
22. The VHH antibody of any one of embodiments 1-20, which is in a multimeric format.
23. The VHH antibody of embodiment 22, which is in a dimeric format.
24.A nucleic acid molecule encoding a VHH antibody according to any one of embodiments 1-23, preferably in operative linkage with a heterologous expression control sequence.
25.A vector comprising a nucleic acid molecule according to embodiment 24.
26. The vector of embodiment 25, which is suitable for integration into the genome of a host cell, e.g., by homologous recombination, transposon-mediated integration or lentiviral integration.
27.A recombinant cell or non-human organism transformed or transfected with a nucleic acid molecule of embodiment 24 or a vector of embodiment 25 or 26.
28. The recombinant non-human organism of embodiment 27 which is a probiotic microorganism secreting a VHH antibody of any one of embodiments 1-23 and which is capable of inhabiting a mammalian, e.g., human mucosa, for example of the female genital tract.
29. The recombinant non-human organism of embodiment 28 which is a non-pathogenic bacterium, for example a Lactobacillus species, such as *L*. *crispatus*, or a yeast.
30.A pharmaceutical composition comprising the VHH antibody of any one of embodiments 1-23, the nucleic acid molecule of embodiment 24, the vector of embodiment 25 or 26 or the cell or organism of any one of embodiments 27-29 and a pharmaceutically acceptable carrier.
31. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for use in medicine, particularly for use in therapy or diagnostics.
32. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for use in the prevention, treatment and/or mitigation of a condition caused by, associated with and/or accompanied by an infection with HSV-1 or HSV-2.
33. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for the use of embodiment 32, wherein the condition is selected from a mucocutaneous infection, an oropharyngeal infection, an ocular infection, and an infection of the central nervous system including meningitis and encephalitis.
34. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for the use of embodiment 32 or 33 wherein the condition is an infection associated with childbirth or with organ transplantation.
35. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for the use of any one of embodiments 31-34 in a human subject.
36. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for the use of embodiment 34 or 35 wherein the subject is selected from an immunocompromised subject including a subject suffering from an HIV infection, e.g., an AIDS patient, a subject suffering from cancer or a subject suffering from an autoimmune disease.
37.The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for the use of embodiment 34 or 35 wherein the subject is an organ transplant patient or a newborn.
38. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for use as a monotherapy.
39. The VHH antibody of any one of embodiments 1-23, the nucleic acid molecule of embodiment 24, the vector of embodiment 25 or 26, the cell or organism of any one of embodiments 27-29 or the pharmaceutical composition of embodiment 30 for use as a combination with at least one further therapy.
40. The VHH antibody of any one of embodiments 1-23, the nucleic acid molecule of embodiment 24, the vector of embodiment 25 26, the cell or organism of any one of embodiments 27-29 or the pharmaceutical composition of embodiment 30 for the use of embodiment 38, wherein the further therapy comprises administration of a herpesvirus DNA polymerase inhibitor.
41. A method for the prevention, treatment and/or mitigation of a condition caused by, associated with and/or accompanied by an infection with HSV-1 or HSV-2 comprising administering an effective dose of the VHH antibody of any one of embodiments 1-23, the nucleic acid molecule of embodiment 24, the vector of embodiment 25 26, the cell or organism of any one of embodiments 27-29 or the pharmaceutical composition of embodiment 30 to a subject in need thereof, particularly to a human subject.
42.A non-infectious, VHH-fusion-blocked HSV-1 or HSV-2 viral particle for use as an immunogen for generating a VHH antibody specifically recognizing a pre-fusion state of a herpesvirus gB polypeptide.

### Description of the Invention

The present invention provides an VHH antibody directed against a pre-fusion-specific conformation of the herpesvirus polypeptide gB. In certain embodiments, the VHH antibody is a neutralizing VHH antibody. The neutralizing activity of the VHH antibody is based on the recognition of a structural epitope that is only available on the surface of the gB protein in pre-fusion form. By this, only the conformation able to perform the fusion function (pre-fusion) is targeted.

Generating VHH antibodies directed against the pre-fusion faced several challenges, the first being to produce a suitable immunogen. Due to the metastable nature of gB, purification of the full-length protein using detergent or as constructs, lacking the transmembrane region, results in gB irreversibly transforming into the post-fusion conformation. An alternative is purification of gB in extracellular vesicles released from transfected cells that overexpress gB (Zeev-Ben-Mordehai *et al*, 2014). On the surface of these vesicles, gB is found in pre- and post-fusion conformation.

For generating VHH antibodies against pre-fusion gB, alpacas were immunised four times with such purified extracellular gB vesicles. A 100 ml blood sample was then taken, lymphocytes recovered, RNA extracted and reverse-transcribed with IgG-specific primers into cDNA, VHH antibody-coding sequences were amplified by nested PCR and ligated into a phage display vector such that they were fused in frame with the pIII M13 protein. Transformation of *E.coli* yielded a library with ~1 billion independent clones. To initiate phage production, bacteria were infected with a helper phage. Obtained phages were then used in phage display, using gB vesicles as baits that were biotinylated with NHS-[PEG]11-Biotin. After two rounds of selection, enriched VHH antibody sequences were again PCR-amplified and cloned into a bacterial expression vector. -200 clones were sequenced and classified by sequence. Finally, 17 candidates were produced as His₁₄-NEDD8 fusions and purified by capture to Ni(II) chelate beads, followed by elution with the tag-cleaving NEDP1 protease (Frey & Gorlich, 2014a) (Pleiner *et al*, 2015).

The obtained VHH antibodies were tested for their ability to neutralize HSV-1. The set of 17 different VHH antibodies revealed a single candidate with potent neutralization capacity. It appears to be a rare VHH antibody, as its sequence was found just once amongst the ~200 clones sequenced, and it was the only representative of its class.

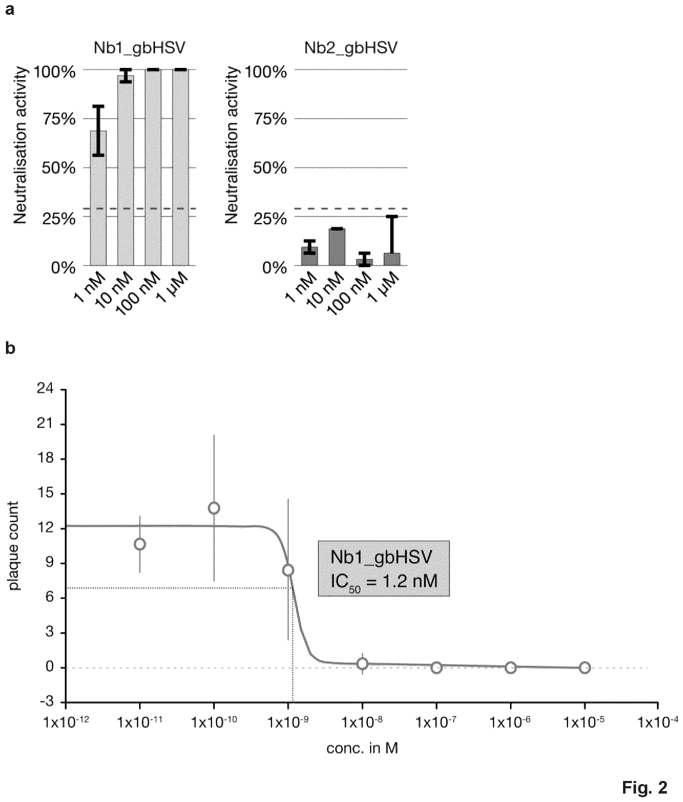

Further analysis of this Nb, called Nb1_gbHSV, revealed an IC₅₀ of 1.2 nM in plaque reduction assays (Figure 2b). All other analyzed anti-gB VHH antibodies showed either no neutralization (exemplified by Nb2_gbHSV) or neutralized only partially and at far higher (µM) concentrations. This emphasizes that obtaining a potent neutralizer directed against the fusion protein was a non-trivial discovery.

Subsequently, differential scanning fluorimetry (DSF) showed Nb1_gBHSV to be stable in the complete temperature range tested (20°C to 95°C), provided, the structural disulfide bond had been formed.

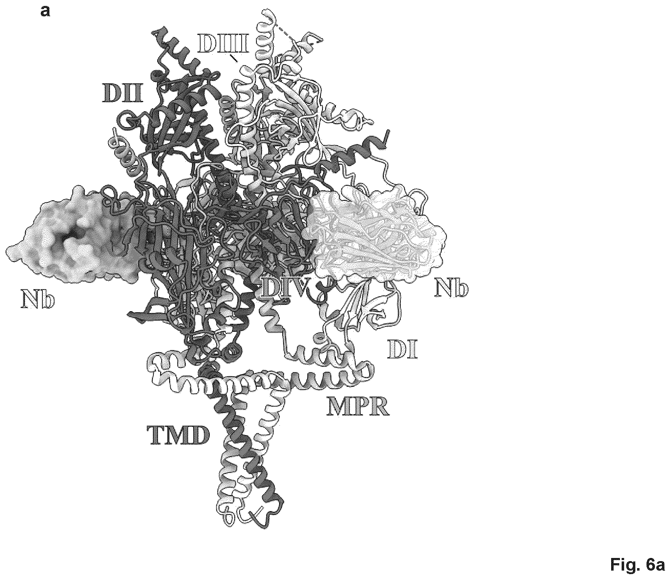

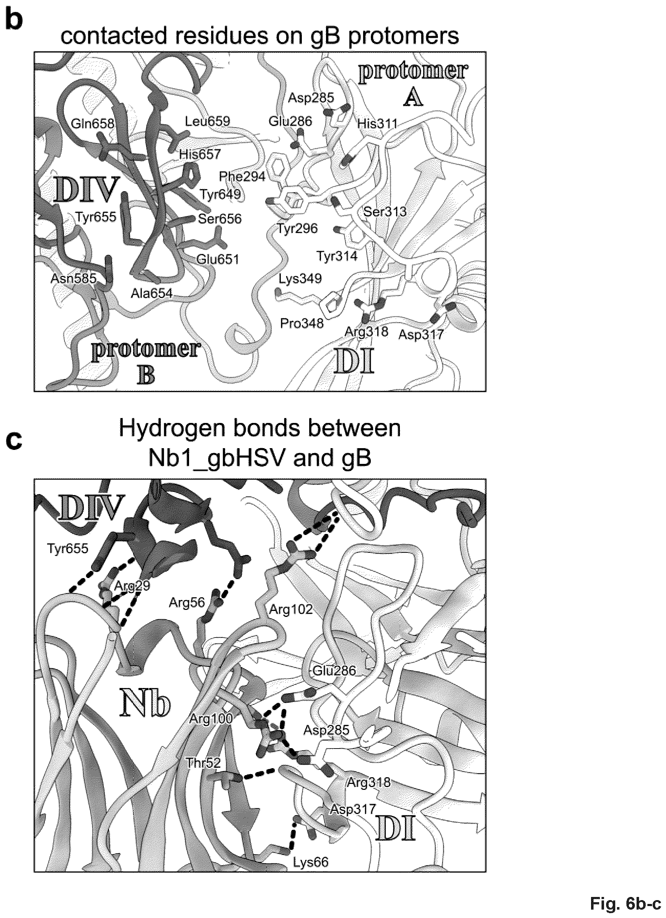

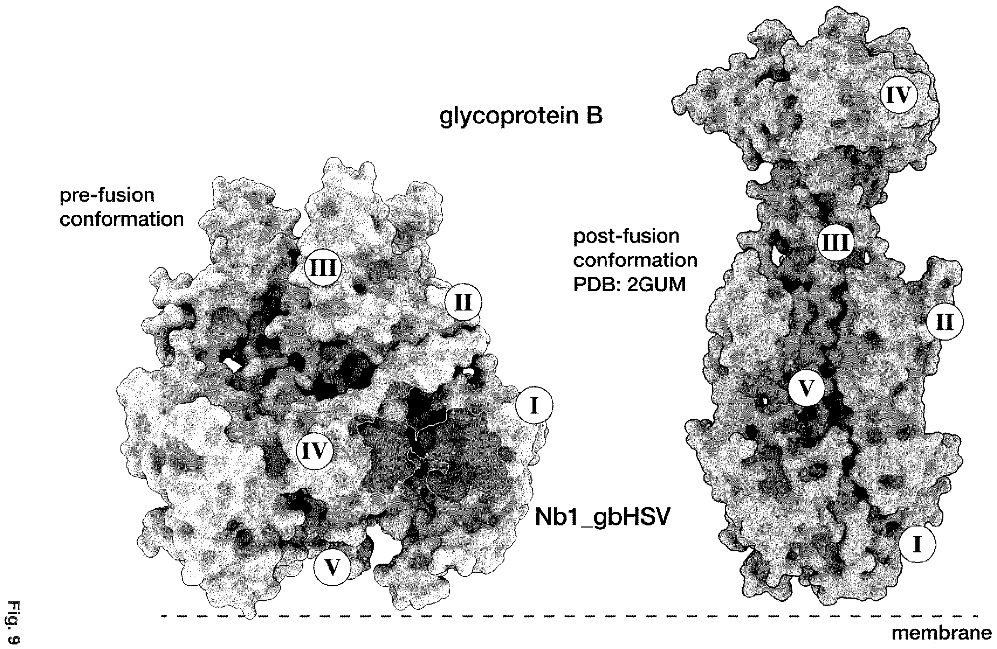

The inventors then bound Nb1_gbHSV to a stabilized mutant of gB, and determined the structure of the resulting nanobody-bound gB trimer by single particle cryo-electron microscopy (Figure 6). This revealed a conformational epitope, distributed over domains I, III, and IV from two adjacent protomers (domain definition as described in (Heldwein *et al*, 2006)). Several hydrogen bonds and salt bridges are formed between Nb1_gbHSV and gB (Figure 6) generating a complex interaction network between the two proteins (Table 1). Two domains (domain I and IV), which contribute most of the interface, are in close proximity in the pre-fusion conformation but are separated by >10 nm in the post-fusion conformation (Figure 9). Furthermore, docking a bound Nb1_gbHSV into a post-fusion structure revealed substantial steric clashes. Thereby, the bound VHH antibody prevents the transition from the pre-fusion to the post-fusion state and thus blocks membrane fusion and infection.

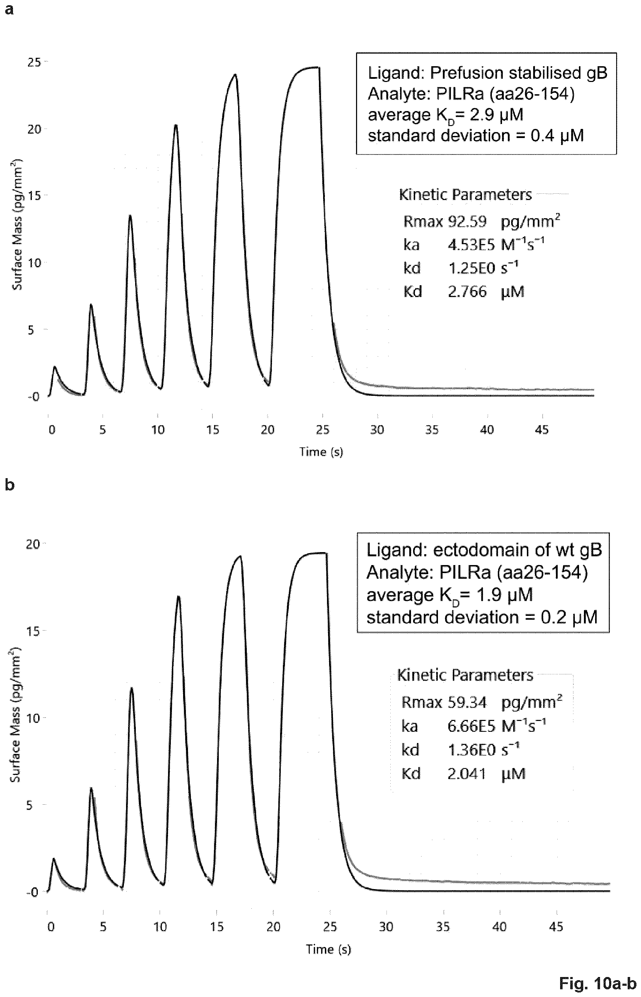

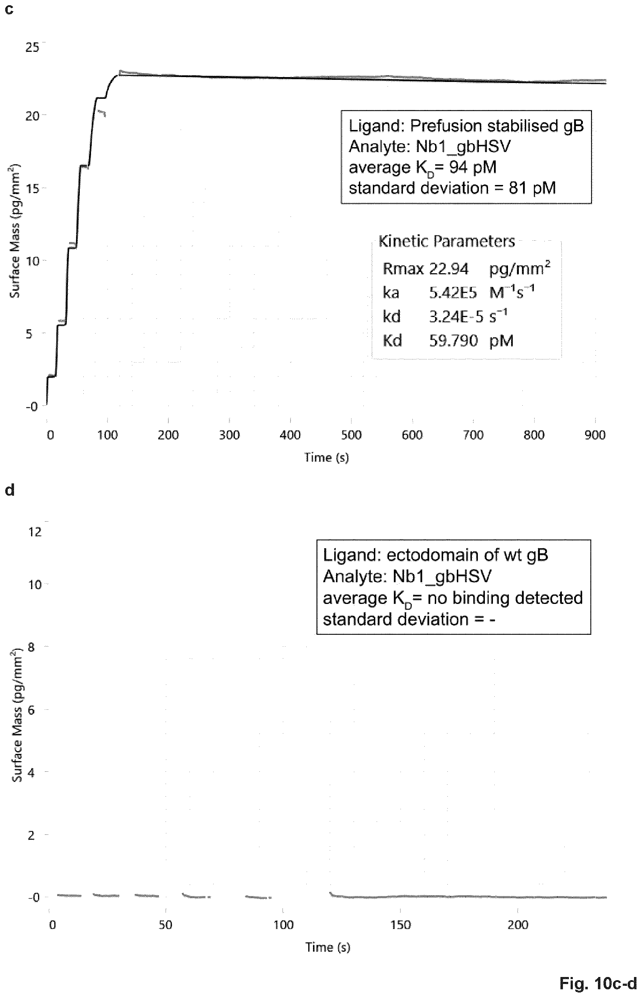

The specificity of Nb1_gbHSV to the pre-fusion conformation of gB was further verified by grating-coupled interferometry (GCI). For this, the inventors immobilised either prefusion stabilised gB or a wild type ectodomain construct that adopts the postfusion conformation via C-terminal strep2 tags. When, as a control, a minimal binding construct of the gB binding receptor PILR*α* (aa26-154) was used as analyte, an average binding affinity KD = 2.9 µM for pre-fusion and KD = 1.9 µM for post-fusion gB was measured. For Nb1_gbHSV the average affinity to pre-fusion was determined in three measurements to be KD of 20 to 200 pM, particularly about 100 pM while no binding was detected for post-fusion gB, further underlining the pre-fusion specificity of Nb1_gbHSV (Figure 10).

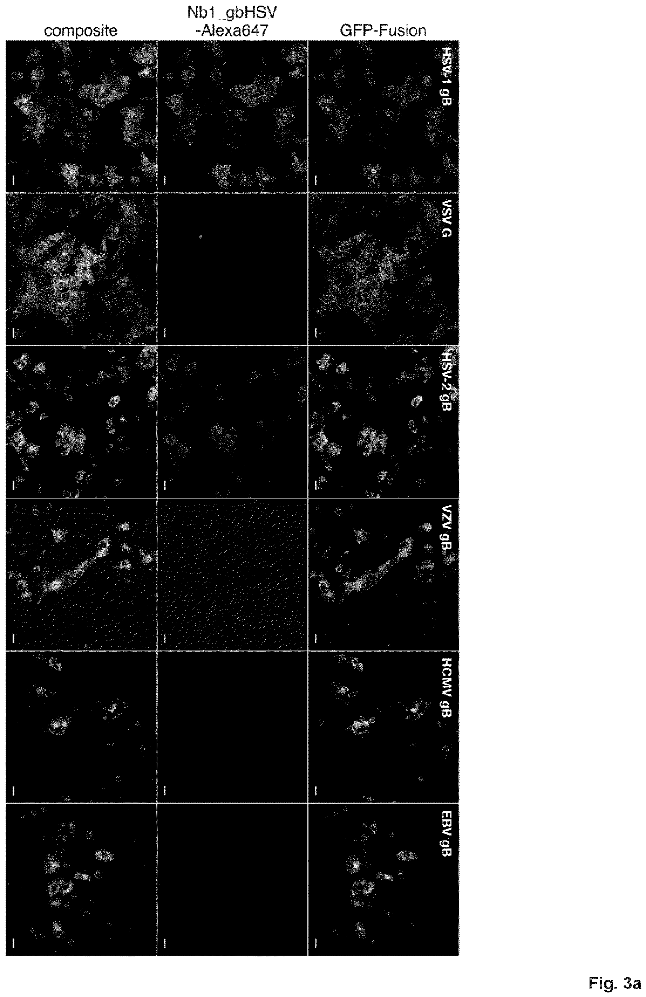

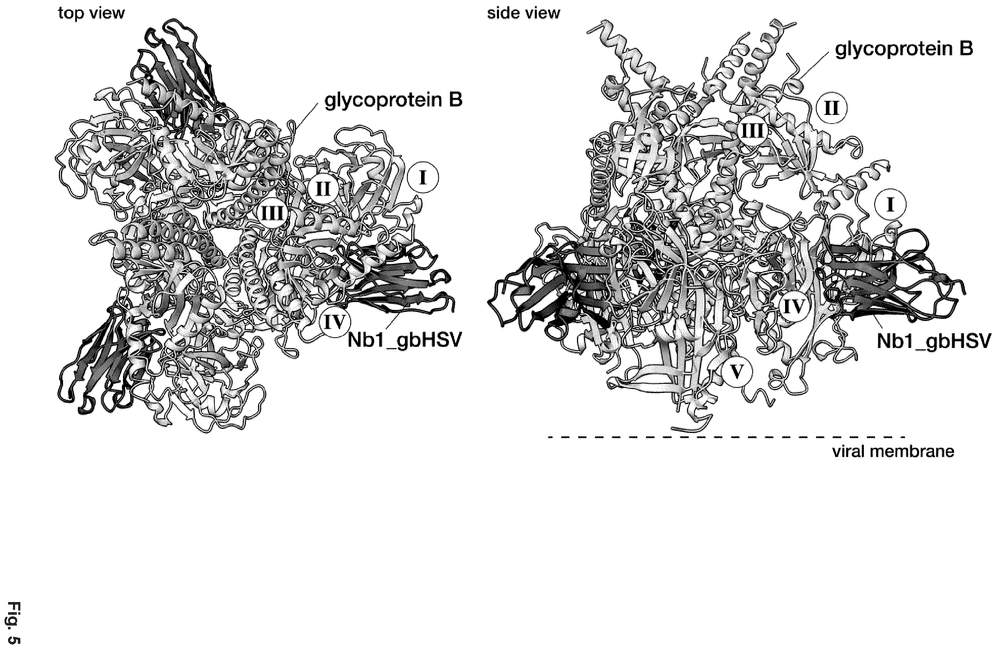

The epitope is highly conserved in HSV-2 gB. Nb1_gbHSV therefore also interacts with this gB homologue as shown via fluorescence microscopy using a fluorescently labelled variant of Nb1_gbHSV on cells transfected with HSV-2 gB (Figure 3a). In addition, the interaction was verified by another cryo-EM structure of Nb1_gbHSV bound to HSV-2 prefusion gB (Figure 5).

### Antibodies

The present invention relates to a VHH antibody targeting the pre-fusion state of a herpesvirus gB polypeptide and inhibiting transition into the post-fusion state.

A VHH antibody according to the present invention is characterized by (i) a CDR3 sequence, (ii) a combination of a CDR1 sequence, a CDR2 sequence, and a CDR3 sequence, (iii) by a complete VHH sequence, and/or (iv) by competition with a specific reference antibody. Specific CDR and VHH sequences are provided herein.

A VHH antibody may be a monovalent heavy chain-only antibody comprising a CDR1 domain, a CDR2 domain and a CDR3 domain linked by framework regions including, but not being limited to, whole VHH antibodies, e.g., native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as Kabat, Chothia, and IMGT. A selected set of CDRs may include sequences identified by more than one method, namely, some CDR sequences may be determined using Kabat and some using IMGT, for example. According to some embodiments of the present invention, the CDR sequences of a variable region are determined using the Kabat method. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

The present invention is also directed to a covalent or non-covalent conjugate of an antibody molecule, e.g., a VHH antibody molecule to a to a heterologous moiety, which may be a non-proteinaceous structure or a heterologous polypeptide moiety. For example, the heterologous moiety may be a label, a capture group such a solid phase-binding group, or an effector.

In certain embodiments, the heterologous moiety is selected from a fluorescence group, biotin, an enzyme such as a peroxidase, phosphatase, or luciferase, a hapten, an affinity tag, or a nucleic acid such as an oligonucleotide. In certain embodiments, the heterologous moiety is conjugated to the C-terminus of an antibody chain or a VHH antibody. These conjugates are e.g., suitable for diagnostic applications.

In particular embodiments, the heterologous moiety is an immunoglobulin Fc sequence. In certain embodiments, the immunoglobulin Fc sequence is a human Fc sequence, particularly a human IgG Fc sequence, e.g., a human IgG1, IgG2, IgG3 or IgG4 sequence.

According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which competes with a specific VHH antibody disclosed herein for the binding to the pre-fusion state of an HSV-1 and/or HSV-2 polypeptide. In certain embodiments, a competing VHH antibody binds the same or an overlapping epitope on the gB polypeptide. For example, the invention refers to a VHH antibody, which competes with the reference antibody Nb1_gbHSV having a VHH sequence as shown in SEQ. ID NO: 13 for the binding to the pre-fusion state of an HSV-1 and/or HSV-2 polypeptide.

Competition may be determined by a cross-competition or epitope binning assay using a label-free detection system, or by fluorescence staining performed as shown in Figure 3a. The signal can be competed with a non-labeled VHH, provided it binds to the same or an overlapping epitope.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In certain embodiments, the VHH antibody is a humanized VHH variant which differs from the original VHH sequence by at least one framework mutation, e.g., by 1 to 15 or 1 to 10 framework mutations, wherein the sequence identity to a human immunoglobulin variable region, e.g., a human germline immunoglobulin variable region is increased. In particular embodiments, a humanized VHH variant comprises humanized framework regions wherein at least one amino acid, e.g.,1 to 10 amino acids in the framework regions, i.e., the regions outside the hypervariable CDR1, CDR2 and CDR3 regions are replaced by other amino acids found in a human framework region.

Methods of humanizing antibody framework sequences are well known in the art as described in a review article by Rossotti et al. (FEBS J. 289 (2022), 4304-4327) and the citations listed therein, the contents of which are herein incorporated by reference. The humanization changes the framework but keeps the paratope intact. In particular embodiments, the combination of CDR1, CDR2 and CDR3 sequences remains unaltered. Thus, a humanized VHH antibody variant of the present invention may comprise a combination of CDR1, CDR2 and CDR3 sequences of the respective camelid VHH antibody and humanized framework regions.

In particular embodiments, a humanized VHH antibody variant of the present invention may comprise a VHH sequence as described herein wherein at least one and up to 15 or up to 10 amino acids of the original camelid framework sequence are replaced by amino acids present at a corresponding position in a framework sequence of a variable domain of a human antibody heavy chain, particularly of a human heavy IgG chain, e.g., a germline version of a variable domain of a heavy IgG chain. Tolerability of individual amino acid replacements at individual positions may be determined in a straightforward manner by testing the characteristics of the VHH antibody variants as described herein below in Example 3 (Virus neutralization), Example 4 (Prevention of virus infection), Example 5 (Recognition of gB polypeptide), Example 6 (Thermostability), Example 7 (Binding epitope determination) and Example 8 (Binding affinity determination). A first test can also be done computationally on the basis of the here disclosed EM structure of the VHH-gB complex.

In further particular embodiments, a humanized VHH antibody variant of the present invention comprises a combination of CDR1, CDR2 and CDR3 sequences as shown in SEQ. ID NO: 14-16 and has an overall amino sequence identity of at least 80%, at least 90% or at least 95% and up to 99% to the VHH sequence of the VHH antibody Nb1_gbHSV as shown in SEQ. ID NO: 13.

In further particular embodiments, the VHH antibody is selected from antibody Nb1_gbHSV having a VHH sequence as shown in SEQ. ID NO: 13 or a VHH antibody, which is a variant thereof including a humanized VHH variant. In certain embodiments, 1, 2, 3, 4, 5, 6, 7, 8, 9 or 10 amino acids of SEQ. ID NO: 13 are replaced by another amino acid.

The present invention also refers to a non-neutralizing VHH antibody specifically recognizing the gB polypeptide of HSV-1 and/or HSV-2. This antibody is useful for diagnostic applications as described below. In particular embodiments, the non-neutralizing VHH antibody comprises a combination of CDR1, CDR2 and CDR3 sequences as shown in SEQ. ID NO: 18-20 and has an overall amino sequence identity of at least 80%, at least 90% or at least 95% and up to 99% to the VHH sequence of the e VHH antibody Nb2_gbHSV as shown in SEQ. ID NO: 17. In further particular embodiments, the non-neutralizing VHH antibody is selected from antibody Nb2_gbHSV having a VHH sequence as shown in SEQ. ID NO: 17 or a VHH antibody, which is a variant thereof including a humanized VHH variant. In certain embodiments, 1, 2, 3, 4, 5, 6, 7, 8, 9 or 10 amino acids of SEQ. ID NO: 17 are replaced by another amino acid.

Further, the present invention relates to a nucleic acid molecule, e.g., a DNA molecule, encoding an antibody, e.g., a VHH antibody as indicated above, or a vector, comprising said nucleic acid molecule as indicated above in operative linkage with an expression control sequence, particularly with a heterologous expression control sequence.

Furthermore, the invention relates to a cell or a non-human organism comprising a nucleic acid molecule or a vector as described above. In certain embodiments, the cell or organism is a probiotic microorganism secreting a VHH antibody as described above and being capable of inhabiting a mammalian, e.g., human mucosa, for example of the female genital tract. In certain embodiments, the recombinant non-human organism may be a non-pathogenic bacterium, for example a Lactobacillus species, such as L. crispatus, or a yeast.

### Binding to the pre-fusion state of HSV-1 and HSV-2 gB polypeptide

The VHH antibody of the invention specifically recognizes a gB polypeptide from HSV-1 and HSV-2 in its pre-fusion state. The amino acid sequence of an HSV-1 gB polypeptide is e.g., described under the accession number UniProt P06437 and the amino acid sequence of an HSV-2 gB polypeptide is e.g., described under the accession number UniProt P06763. The VHH antibody of the invention may also bind to sequence variants of UniProt P06437 and UniProt P06763 derived from different HSV-1 or HSV-2 strains.

According to certain embodiments, the gB polypeptide from HSV-1 comprises amino acids 31-904 of the amino acid sequence shown in SEQ ID NO: 1 (i.e. the amino acid sequence of a mature HSV-1 gB polypeptide) or a corresponding amino acid sequence from a different HSV-1 strain having an identity of at least 95%, at least 98% or at least 99% thereto over the whole length of the mature polypeptide.

In particular embodiments, the VHH antibody of the invention binds to a conformational epitope on the HSV-1 gB polypeptide comprising 27 amino acids A106, N108, G121, A122, D285, E286, G292, F294, Y296, H311, T312, S313, Y314, A315, D317, P348, K349, N585, Y640, E651, A654, Y655, S656, H657, Q658, L659, and D663 of SEQ ID NO: 1 wherein D285 may be replaced by N285 and/or S312 may be replaced by T313 or an epitope comprising at least 17 or at least 22 amino acids thereof.

According to certain embodiments, the gB polypeptide from HSV-2 comprises amino acids 23-904 of the amino acid sequence shown in SEQ ID NO: 2 (i.e. the amino acid sequence of a mature HSV-2 gB polypeptide) or a corresponding amino acid sequence from a different HSV-2 strain having an identity of at least 95%, at least 98% or at least 99% thereto over the whole length of the mature polypeptide.

In particular embodiments, the VHH antibody of the invention binds to a conformational epitope on the HSV-2 gB polypeptide comprising 24 amino acids V101, G116, A117, D280, E281, G287, F289, Y291, H306, T307, S308, Y309, A310, R313, P343, N582, Y637, E648, A651, Y652, S653, H654, Q655 and D660 of SEQ ID NO: 2 wherein S308 may be replaced by T308 or an epitope comprising at least 14 or at least 19 amino acids thereof.

In certain embodiments, the VHH antibody has a binding affinity to a gB polypeptide from HSV-1 in its pre-fusion state expressed as dissociation constant KD of about 1 nM or less, about 0.5 nM or less, about 0.2 nM or less, or about 0.1 nM or less when measured as described in the Examples in detail.

In certain embodiments, the VHH antibody does not bind to a gB polypeptide from HSV-1 and HSV-2 in its post-fusion state. In certain embodiments, the VHH antibody has a binding affinity to a gB polypeptide from HSV-1 in its post-fusion state expressed as dissociation constant KD of about 1 µM or higher, 10 µM or higher or 100 µM or higher when measured as described in the Examples in detail.

### Neutralization

The VHH antibody of the invention is capable of neutralizing HSV-1. The neutralization capacity describes the potency of an anti-HSV-1 agent and may be determined in a plaque reduction assay on Vero cells (ATCC CCL-81) as described in Examples 3 and 4 and Figures 2a and 2b.

In certain embodiments, the VHH antibody has a neutralization capacity expressed as IC₅₀ of about 10 nM or less, about 5 nM or less, or about 1.5 nM or less. For the preferred VHH antibody Nb1_gbHSV an IC₅₀ of 1.2 nM was found.

### Stability

For the intended therapeutic application, the VHH antibodies should not only be highly potent in herpesvirus neutralization, but also, they should be developable as biological drugs. This includes that they are stable enough to survive a lengthy, large-scale production process as well as transportation and storage (ideally for years in liquid formulation) without aggregation or loss of activity.

This also includes that the VHH antibodies should be stable when produced in the cytoplasm of probiotic microorganism secreting the antibodies.

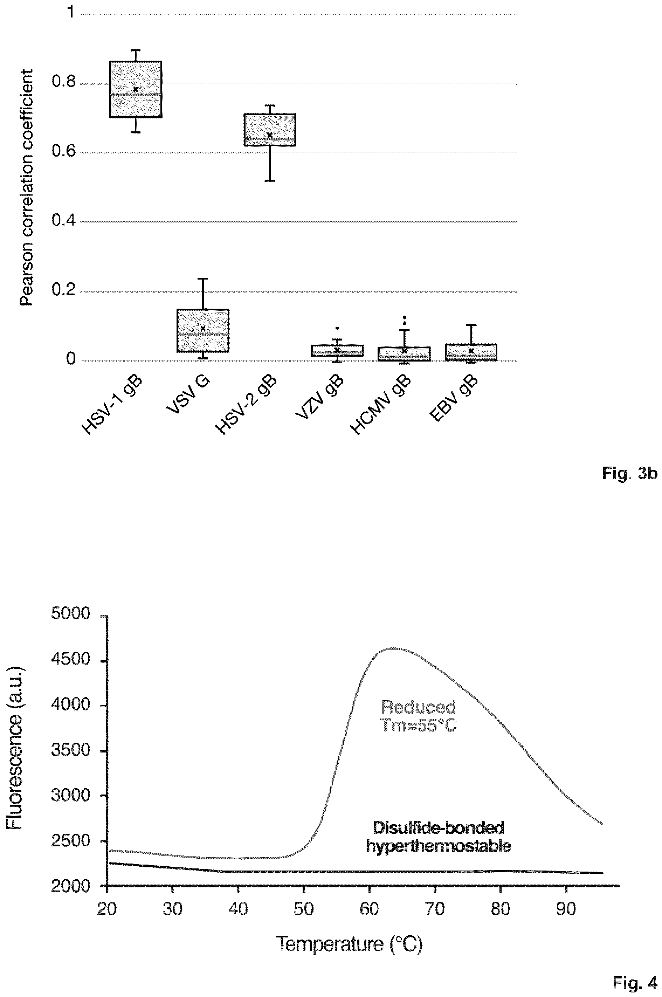

A good predictor for stability is thermostability, which can be measured, e.g., by thermal shift assays or specifically by differential scanning fluorimetry. The present inventors have found that the VHH antibody Nb1_gbHSV in its fully disulfide-bonded form is hyperthermostable (melting temperature of at least 95°C) as shown in Example 6 and Figure 4.

A method of producing a stable VHH antibody in its fully-disulfide bonded form is described in WO 2024/256467, the content of which is herein incorporated by reference. This method encompasses the steps:
(i) expressing a VHH antibody comprising two structural cysteines and at least one solvent-accessible ectopic cysteine in a host cell under reducing or at least partially reducing conditions,
(ii) purifying the VHH antibody obtained in step (i) from the host cell or the culture medium under conditions wherein the at least one solvent-accessible ectopic cysteine is in the reduced SH state,
(iii) optionally attaching a labelling agent to the at least one solvent-accessible ectopic cysteine under conditions while keeping the two structural cysteines unmodified,
(iv) subjecting the VHH antibody obtained in step (ii) or (iii) to an oxidation that converts the two structural cysteines to a disulfide bond, and
(v) obtaining a thermostabilized VHH antibody wherein the two structural cysteines are quantitatively oxidized to a disulfide bond.

In a further embodiment, a fully disulfide-bonded VHH antibody may be obtained by periplasmic production in a bacterium, e.g., E.coli, or secretory production in a eukaryotic cell, e.g., a yeast or mammalian cell.

In a particular embodiment, the invention relates to a VHH antibody, which is stable, particularly thermostable, or hyperthermostable. Preferably, the VHH antibody has a melting temperature of at least about 40°C, of at least about 50°C, or of at least about 55°C, when measured under reducing conditions and/or a melting temperature of at least about 60°C, of at least about 80°C, of at least about 90°C, or of at least about 95°C when measured under non-reducing conditions. Melting temperatures are determined as described herein.

### Monovalent and multivalent antibodies

In certain embodiments, the VHH antibody of the present invention is in a monovalent format, i.e., it has a single binding site for the gB polypeptide. In these embodiments, the antibody may be present as such or covalently or non-covalently attached to a heterologous moiety, e.g., a peptidic or non-peptidic moiety.

In further embodiments, the antibody of the present invention is in a multimeric, e.g., dimeric, or trimeric format. In these embodiments, several VHH antibody units may be covalently or non-covalently attached to each other together via a linker and/or a multimerization, e.g., dimerization or trimerization moiety.

In certain embodiments, the antibody may be covalently or non-covalently conjugated to a heterologous moiety, which is selected from a labeling group, a capture group, or an effector group, and wherein the heterologous moiety is particularly selected from a fluorescence group, biotin, an enzyme such as a peroxidase, phosphatase, or luciferase, a hapten, an affinity tag, or a nucleic acid such as an oligonucleotide. In further embodiments, the heterologous moiety is selected from human serum albumin, an albumin-binding moiety, or an Fc fragment of an immunoglobulin molecule, e.g., IgA, IgD, IgE, IgG, IgM, or a subtype thereof. In still further embodiments, the heterologous moiety is selected from one or several non-peptidic polymer moieties, preferably hydrophilic polymer moieties, such as polyethylene glycol (PEG).

In particular embodiments, the VHH antibody is a homodimeric VHH antibody, wherein a VHH antibody unit is covalently attached to a dimerization moiety, e.g., an immunoglobulin IgG Fc fragment, particularly a human IgG Fc fragment.

In certain embodiments, the immunoglobulin IgG Fc fragment has an effector function which may mediate cytotoxic effects. The effector function may comprise binding to an Fc receptor, e.g., an FcyR or CD16, and/or recruiting complement proteins.

### Diagnostic applications

There are different possible applications for which the VHH antibodies of the present invention, e.g., VHH antibodies Nb1_gbHSV or Nb2_gbHSV are suitable, but primarily as therapeutic drug and/or diagnostic tool.

The latter can be used to detect herpesviruses in biological samples, e.g., patient samples by an immunological assay such as an ELISAs or a lateral flow test. For these applications, the VHH antibody can be linked to various labels including fluorescent labels, biotin or enzymes, and/or it can be surface immobilized. Similarly, detection of the gB protein using the VHH antibody can also be used in experimental applications like fluorescence microscopy of infected or transfected living cells as well as chemically fixed cells.

### Therapeutic applications

The VHH antibody disclosed herein, a nucleic acid molecule encoding the antibody or a cell or non-human organism comprising the nucleic acid molecule can be used for the prevention, mitigation and/or treatment of a condition caused by, associated with and/or accompanied by an infection with HSV-1 or HSV-2, particularly in a human subject.

For therapeutic applications, the active agent, i.e., the VHH antibody, a nucleic acid molecule encoding the antibody or a cell or non-human organism comprising the nucleic acid molecule, may be provided as a pharmaceutical composition comprising the active agent and a pharmaceutically acceptable carrier.

Suitable types of pharmaceutical compositions are known in the art, e.g., as described in Remington, The Science and Practice of Pharmacy 23rd Edition.

The VHH antibody may be administered to a subject who is not yet infected with HSV-1 and/or HSV-2 and may be at risk of being infected. The VHH antibody may also be administered to a subject who is already infected HSV-1 and/or HSV-2 and wherein reactivation of a dormant virus needs to be suppressed or wherein an active virus infection needs to be combatted.

The term "prevention" particularly refers to an administration to a subject who does not suffer from an active infection, e.g. to a subject who is at risk of being newly infected with HSV-1 and/or HSV-2 or who is at risk of reactivation of a dormant infection with HSV-1 and/or HSV-2, i.e., a prophylactic administration. The term "treatment" particularly refers to an administration to a subject who is already suffering from an active infection with HSV-1 and/or HSV-2. The term "mitigation" refers to an alleviation of viral load and optionally signs and symptoms of the infection.

The VHH antibody recognizes a conformational epitope that is hardly accessible for conventional antibodies, whose antigen-binding sites consist of light and heavy chains and that are thus bulkier. This makes the VHH antibody less likely to compete with patients' antibodies and thus to suppress an (a further) antibody response by the patient. It can thus help to boost active immunity against HSV-1 and/or HSV-2 while treating an infection. The nearly absolute conservation of the epitope between HSV-1 and HSV-2 (Table 1) suggests that immune escape by viral mutations in gB is unlikely.

Preferred indications include acute, generalized infections by HSV-1 or HSV-2, where the VHH antibody can block a spreading of the virus, e.g., over longer distances and between organs. It can also eliminate already infected, virus-producing cells and thus reduce the viral load.

In particular embodiments, the condition to be treated, prevented and/or mitigated is selected from a mucocutaneous infection, an oropharyngeal infection, an ocular infection, and an infection of the central nervous system including meningitis and encephalitis. In further particular embodiments, the condition is an infection associated with childbirth or with organ transplantation.

In further particular embodiments, the subject to whom the VHH antibody is administered is selected from an immunocompromised subject including a subject suffering from an HIV infection, e.g., an AIDS patient, a subject suffering from cancer or a subject suffering from an autoimmune disease.

In further particular embodiments, the subject is an organ transplant patient or a newborn.

The preferred route of administration of the VHH antibody is by injection. The injected VHH antibody can then neutralize circulating virus and block the infection of new cells. When fused to an Fc fragment with appropriate effector functions (Bournazos & Ravetch, 2017), the antibody can also eliminate already infected cells by antibody-mediated cytotoxicity. An appropriate Fc fusion will also increase the plasma half-life by slowing down renal excretion and by allowing for an FcRn-(neonatal Fc receptor)-mediated recycling from the primary urine (Pyzik *et al,* 2023). This way, a single injection may provide a therapeutic effect and protection for several months.

Herpes simplex encephalitis is a particularly severe form of HSV infection. It results in haemorrhagic necrosis of the inferomedial region of the temporal lobe; it is associated with high mortality, even with state-of-the-art treatment using polymerase inhibitors; and surviving patients typically suffer from long-term neurological complications (Whitley, 1996). The VHH antibody offers new and potentially more effective treatment options by blocking infection of new cells at the earliest possible step. The increased permeability of the blood-brain barrier during encephalitis (Platt *et al*, 2017) would allow passage of the VHH antibody into the brain. Otherwise, a direct application into the cerebrospinal fluid by means of an intrathecal injection may be considered.

Immunocompromised patients are particularly likely to benefit from an antiviral therapy with the VHH antibody. These include patients receiving an immunosuppressive treatment, patients with genetic immune defects, or HIV-positive patients with AIDS symptoms, who have decreased levels of HSV-specific CD8+ cytotoxic T lymphocytes and thus suffer from more frequent and more severe HSV reactivations (Posavad *et al*, 1997) (Mole *et al,* 1997). The VHH antibody can also be used prophylactically to prevent an HSV infection in the first place.

Another preferred area of application is in transplantation medicine, where transmission of HSV from an organ donor to an organ recipient is a serious complication, especially since the prevention of graft rejection usually requires a long-term immunosuppressive treatment. The use of acyclovir in organ transplant recipients has reduced the risk (Ljungman, 2001), but there is still a risk of developing serious HSV disease (Remeijer *et al*, 2001), also because of the emergence of resistant strains. Furthermore, acyclovir and other viral polymerase inhibitors only affect viral replication. They cannot clear infectious particles or infected cells from the transplants and have a short plasma half-life of only a few hours.

Treating transplant recipients with a VHH antibody of the invention, e.g., Nb1_gbHSV-Fc fusion, will provide several key advantages, namely: (1) the treatment is orthogonal to the nucleoside analogues, (2) the VHH will be effective also against strains that are resistant to polymerase inhibitors, (3) the antibody blocks already the first step of infection, (4) it can eradicate infected cells in the transplant, and (5) the long plasma half-life comes with a long-lasting effect. The treatment is particularly indicated if the donor is HSV-positive, and the recipient is seronegative.

A further application of the VHH antibody of the invention, e.g., Nb1_gbHSV, is the prevention of neonatal herpes, which can lead to a fulminant infection with high mortality and permanent impairments of survivors. A parenteral treatment of the mother with the VHH antibody a Nb1_gbHSV-IgG Fc fusion well before delivery, e.g., at least 2 weeks or at least 4 weeks before the expected delivery can reduce the virus load and thus the risk of transmission. In addition, FcRn-mediated transport across the placenta will supply the foetus with the protective antibody and thus confer immunity to the newborn.

Another embodiment is to supplement the vaginal microbial flora with a probiotic microorganism that secretes an VHH antibody of the invention, e.g., the protective Nb1_gbHSV VHH antibody. This will neutralize HSV in the body fluids and thus reduce the risk of transmission during childbirth. The same strategy can be used for ameliorating the symptoms of herpes in the female genital tract and to reduce the risk of transmission during sexual contacts. The VHH-secreting organism may be an engineered non-pathogenic bacterium, for example a *Lactobacillus* species, such as *L. crispatus*, or a yeast. This treatment would be less invasive than an antibody injection and may yet provide a long-lasting protection.

Topical application of the VHH antibody, e.g., Nb1_gbHSV, also appears feasible for the treatment of HSV infections of the eye, i.e. herpetic keratitis or conjunctivitis. Here, eye drops may be applied that contain the virus-neutralizing VHH antibody.

In principle, therapies with herpesvirus-specific viral DNA polymerase inhibitors including but not limited to acyclovir, ganciclovir, penciclovir, cidofovir and foscarnet, can be combined with the use of the VHH antibody of the invention, e.g., Nb1_gbHSV, to increase efficacy and/or reduce the risk of emergence of antiviral-resistant strains, as these two types of antivirals have completely different modes of action and block different stages of the viral 'life' cycle.

### Brief description of the figures

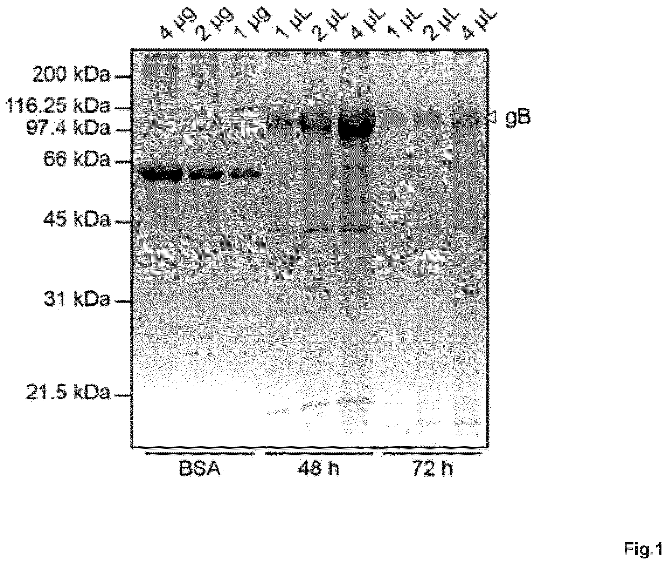

**Figure 1****: Vesicle purification for Alpaca immunisation.**
   SDS PAGE analysis of purified vesicles in comparison with BSA standard. ~3 mg gB protein in vesicles were collected in total for the 48 h sample. 30x T175 flasks of BHK21 cells were transfected using Lipofectamine 2000 with a plasmid coding for HSV-1 gB with the point mutation Y889A to increase formation of extracellular vesicles. Cell supernatants were collected 48h and 72h after transfection and concentrated using a Vivaflow (Sartorius) diafiltration system with a 100 kDa MW Cut-off. Concentrated vesicles in the remaining supernatant were further concentrated by ultracentrifugation at 100,000 x g for 2 h at 4°C through a 20% sucrose cushion. Vesicle pellets were rehydrated overnight at 4°C in ~100 µL PBS + 250 mM sorbitol / T175 flask start material.
**Figure 2****: Plaque reduction assay and IC50 determination.**
   a) A plaque reduction assay allows to measure the potency of an antiviral agent and is a variant of the conventional plaque assay. For this, a confluent monolayer of Vero cells in a 24-well plate was incubated for 1.5 h at room temperature (RT) with media containing 160 plaque forming units per ml (pfu/ml) of HSV-1 before the inoculum was removed and cells were washed with PBS. The cell layer was then covered with an immobilizing semisolid overlay medium (1.2% avicell in DMEM and 5% FBS) to prevent indiscriminate spreading of newly formed virions. The viral infection and replication are therefore constrained to the surrounding cells and individual plaques, or zones of cell death resulting from infection of a single cell are formed after 2-4 days of infection. The remaining cells were then fixed with 4% paraformaldehyde in H₂O and stained using 0.1% crystal violet in 2% ethanol while the dead cells were washed off, leading to countable holes or 'plaques' in the cell layer. To determine the neutralization activity of individual nanobodies, viruses were pre-incubated with the given concentrations of the shown nanobody prior to cell layer infection. Each data point represents the average number of plaques in two separate cell layer infections with HSV-1, normalised to buffer control and depicted as neutralization activity with 100% meaning no plaques and 0% meaning the same or more plaques as counted in the buffer control. Error bars show the complete range. The dashed line shows the range of the buffer control. In comparison to Nb1_gbHSV, which has a high neutralization activity in all tested concentrations, Nb2_gbHSV shows no neutralization activity.
   b) Experiments were performed as described in a). The IC50 value for Nb1_gbHSV was calculated using the AAT Bioquest calculator (https://www.aatbio.com/tools/ic50-calculator). Each data point represents the average number of plaques in three separate cell layer infections with herpes simplex virus 1 of three independent experiments. Error bars show the standard error of the mean. The calculated IC₅₀ of Nb1_gbHSV is 1.2 nM.
**Figure 3****: Cross-reactivity test using Alexa647 labelled Nb1_gbHSV.**
   a) ARPE-19 cells were grown in Ibidi µ-Slide 8 Well chambered coverslips for 24 h before transient transfection using Lipofectamine 2000. Cells were transfected with plasmids for expression of different gB homologues, namely from HSV-1, HSV-2, VZV, HCMV or EBV. In addition, as negative control the plasma membrane resident viral transmembrane glycoprotein from Vesicular Stomatitis Virus was transfected. All constructs were based on the respective wild-type sequence with a C-terminally added fluorescent sfEGFP tag. 1 µg DNA + 1 µL Lipofectamine / well in 200 µL DMEM media. 48 h post transfection, cells were washed with PBS before addition of 200 µL fresh DMEM media including 1 µM of Alexa647 labelled Nb1_gbHSV nanobody. After 1 h incubation at 37°C, cells were washed with PBS and the media was replaced. Cells were imaged live with identical settings. Images were analysed using the FIJI software and the JaCoP plugin. Pearson correlation coefficients of the measured fluorescent signal in the green and red channels were calculated and are shown as box-and-whisker plot. The median value is marked by a horizontal line, while the box marks the upper and lower quartile limits, and the whiskers showing the min and max value. Outliers are marked as points outside the whisker range. Only for HSV-1 and HSV-2 gB the fluorescent signal of the sfEGFP tag correlates with the signal from Nb1_gbHSV.
   b) Example fluorescent images of gB transfected cell stained with the Alexa647 labelled nanobody. While HSV-1 and HSV-2 gB transfected cells show a signal in the Alexa647 channel, EBV gB transfected cells show only background level signal.
**Figure 4****: Thermostability of Nb1_gbHSV.**
   Thermostability was measured by differential scanning fluorimetry (DSF) for Nb1_gbHSV produced with reduced cysteines (grey line) or following formation of the structural disulfide bond (black line) in the depicted temperature range. Unfolding causes an increase in fluorescence (by exposing hydrophobic residues that bind the added SYPRO Orange dye). Melting temperature (Tm) is defined as the inflection point of the melting curve before reaching the first melting peak. Nanobodies are considered hyperthermostable if they produced no melting peak. Reduced Nb1_gbHSV has a Tm of 55°C, while the disulfide-bonded form showed no melting throughout 95°C and is thus hyperthermostable.
**Figure 5****: Structure of pre-fusion HSV-1 gB bound by Nb1_gbHSV.**
   The structure of pre-fusion gB from HSV-1 in complex with Nb1_gbHSV was determined by cryo electron microscopy single particle analysis. Ectodomains of gB are marked by roman numerals I, II, III and IV according to the definition proposed in Heldwein et al.(Heldwein *et al.*, 2006). Anti-gB nanobody Nb1_gbHSV is shown in dark grey, gB is shown in light grey.
**Figure 6****: Structural description of the nanobody interaction with gB.**
   a) The gB structure is shown in ribbon rendering with the bound nanobody in surface rendering. Nb1_gbHSV facing the front is shown with semi-transparent surface revealing the ribbon representation.
   b) Focus on the main contact region on gB in ribbon rendering, not showing the nanobody structure for better visibility. Domain I (DI) and domain IV (DIV) of protomers A and B are marked in light and dark grey respectively. Residues involved in the gB - Nb1_gbHSV interaction are shown as sticks marked in three-letter code.
   c) Ribbon rendering of the nanobody (Nb) bound gB structure, zoomed in on the main interaction site between Nb1_gbHSV and DIV and DI of gB. Residues involved in the gB - Nb1_gbHSV interaction are shown as sticks with hydrogen bonds marked by black, dashed lines and marked in three-letter code.

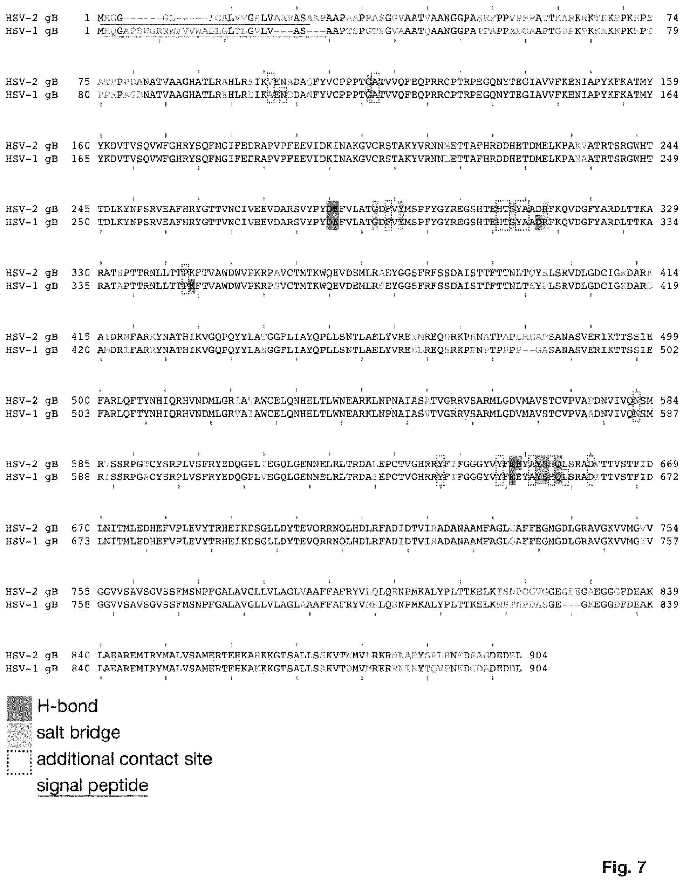

**Figure 7****: Alignment of HSV-1/-2 gB with Nb1_gbHSV epitope.**
   HSV-1 (UniProt: P06437) and HSV-2 (UniProt: P06763) gB sequences were aligned using the UniProt alignment tool (www.uniprot.org). Non-aligning residues are shown in grey. Residues on gB interacting with Nb1_gbHSV forming H-bonds are marked by dark grey backgrounds and residues forming salt bridges are marked by light grey backgrounds. Additional interacting residues are shown in dotted boxes. The signal peptides cleaved during expression and missing from the final protein are underlined.

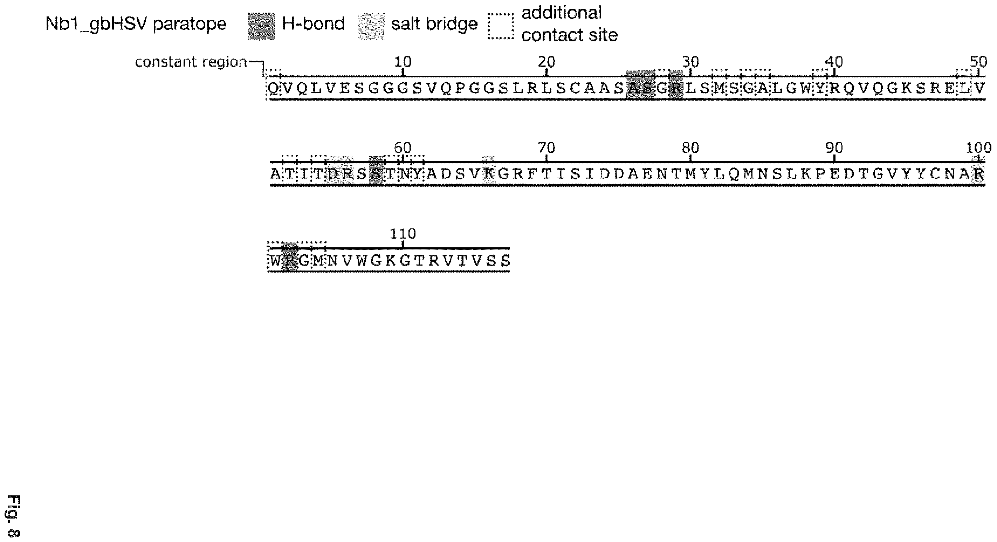

**Figure 8****: Paratope shown on the Nb1_gbHSV sequence (SEQ ID NO: 13).**
   Residue sequence of Nb1_gbHSV is shown with numbering for the constant nanobody sequence. Residues on Nb1_gbHSV interacting with gB forming H-bonds are marked by dark grey backgrounds and residues forming salt bridges are marked by light grey backgrounds. Additional interacting residues are shown in dotted boxes.
**Figure 9****: Domain re-arrangement during membrane fusion.**
   Surface rendering of the pre-fusion conformation structure with the Nb1_gbHSV epitope coloured in dark grey and white outline (left) in comparison with the post-fusion conformation structure determined by X-ray crystallography (Heldwein *et al.,* 2006) (right) to illustrate the position of domains in the two conformations.
**Figure 10****: Conformation specific binding affinity of Nb1_gbHSV to HSV-1 gB.**
   Binding affinities of PILR α (a-b) and nanobody Nb1_gbHSV (c-d) to pre- and postfusion gB were measured using GCI. Prefusion-stabilised (a, c) or postfusion gB (ectodomain of WT) (b, d) were immobilised in respective channels and WaveRAPID measurements were conducted to determine binding affinities using the intermediate (PILR α) or tight (Nb1_gbHSV) binder protocol. Measurements were repeated three times to calculate the average binding affinity (KD) and standard deviation and of which. One representative of each measurement is shown.

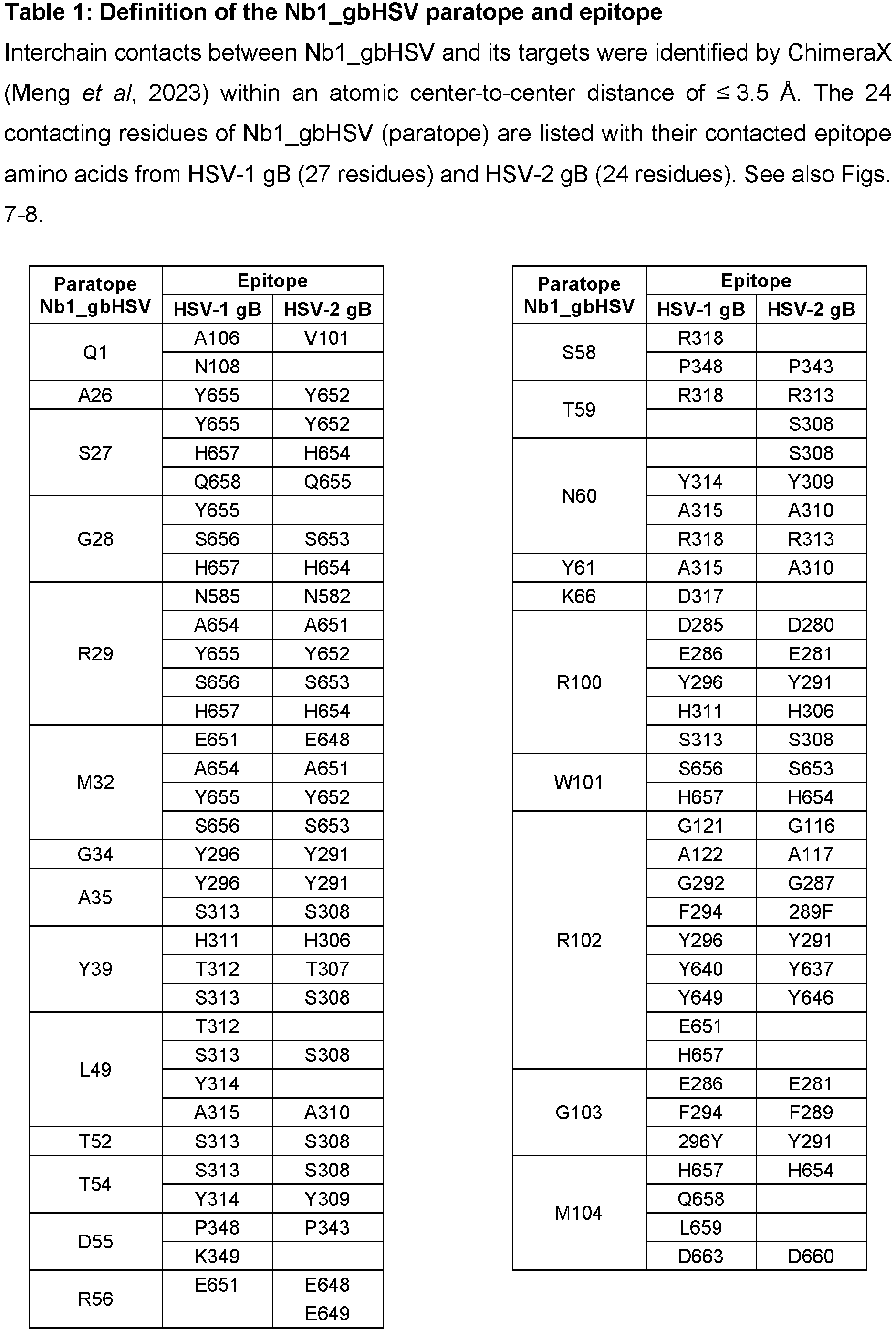

### Sequences of viral proteins

>gB_HSV1 with signal peptide: SEQ ID NO: 1 (amino acids 1-904)
MHQGAPSWGRRWFVVWALLGLTLGVLVASAAPTSPGTPGVAAATQAANGGPATP
APPPLGAAPTGDPKPKKNKKPKNPTPPRPAGDNATVAAGHATLREHLRDIKAENTD
ANFYVCPPPTGATVVQFEQPRRCPTRPEGQNYTEGIAVVFKENIAPYKFKATMYYK
DVTVSQVWFGHRYSQFMGIFEDRAPVPFEEVIDKINAKGVCRSTAKYVRNNLETTA
FHRDDHETDMELKPANAATRTSRGWHTTDLKYNPSRVEAFHRYGTTVNCIVEEVD
ARSVYPYDEFVLATGDFVYMSPFYGYREGSHTEHTSYAADRFKQVDGFYARDLTT
KARATAPTTRNLLTTPKFTVAWDWVPKRPSVCTMTKWQEVDEMLRSEYGGSFRFS
SDAISTTFTTNLTEYPLSRVDLGDCIGKDARDAMDRIFARRYNATHIKVGQPQYYLA
NGGFLIAYQPLLSNTLAELYVREHLREQSRKPPNPTPPPPGASANASVERIKTTSSIE
FARLQFTYNHIQRHVNDMLGRVAIAWCELQNHELTLWNEARKLNPNAIASVTVGRR
VSARMLGDVMAVSTCVPVAADNVIVQNSMRISSRPGACYSRPLVSFRYEDQGPLV
EGQLGENNELRLTRDAIEPCTVGHRRYFTFGGGYVYFEEYAYSHQLSRADITTVST
FIDLNITMLEDHEFVPLEVYTRHEIKDSGLLDYTEVQRRNQLHDLRFADIDTVIHADAN
AAMFAGLGAFFEGMGDLGRAVGKVVMGIVGGVVSAVSGVSSFMSNPFGALAVGLL
VLAGLAAAFFAFRYVMRLQSNPMKALYPLTTKELKNPTNPDASGEGEEGGDFDEAK
LAEAREMIRYMALVSAMERTEHKAKKKGTSALLSAKVTDMVMRKRRNTNYTQVPN
KDGDADEDDL

Amino acids 1-30 corresponding to the N-terminal signal peptide sequence are indicated in italics. 27 amino acids defining the epitope recognized by the antibody Nb1 are indicated in bold letters and underlined:
A106, N108, G121, A122, D285, E286, G292, F294, Y296, H311, T312, S313, Y314, A315, D317, P348, K349, N585, Y640, E651, A654, Y655, S656, H657, Q658, L659 and D663.

>gB_HSV-2 with signal peptide: SEQ ID NO: 2 (amino acids 1-904)
MRGGGLICALVVGALVAAVASAAPAAPAAPRASGGVAATVAANGGPASRPPPVPS
PATTKARKRKTKKPPKRPEATPPPDANATVAAGHATLRAHLREIKVENADAQFYVC
PPPTGATVVQFEQPRRCPTRPEGQNYTEGIAVVFKENIAPYKFKATMYYKDVTVSQ
VWFGHRYSQFMGIFEDRAPVPFEEVIDKINAKGVCRSTAKYVRNNMETTAFHRDDH
ETDMELKPAKVATRTSRGWHTTDLKYNPSRVEAFHRYGTTVNCIVEEVDARSVYPY
DEFVLATGDFVYMSPFYGYREGSHTEHTSYAADRFKQVDGFYARDLTTKARATSP
TTRNLLTTPKFTVAWDWVPKRPAVCTMTKWQEVDEMLRAEYGGSFRFSSDAISTT
FTTNLTQYSLSRVDLGDCIGRDAREAIDRMFARKYNATHIKVGQPQYYLATGGFLIA
YQPLLSNTLAELYVREYMREQDRKPRNATPAPLREAPSANASVERIKTTSSIEFARL
QFTYNHIQRHVNDMLGRIAVAWCELQNHELTLWNEARKLNPNAIASATVGRRVSAR
MLGDVMAVSTCVPVAPDNVIVQNSMRVSSRPGTCYSRPLVSFRYEDQGPLIEGQL

GENNELRLTRDALEPCTVGHRRYFIFGGGYVYFEEYAYSHQLSRADVTTVSTFIDLN
ITMLEDHEFVPLEVYTRHEIKDSGLLDYTEVQRRNQLHDLRFADIDTVIRADANAAMF
AGLCAFFEGMGDLGRAVGKVVMGVVGGVVSAVSGVSSFMSNPFGALAVGLLVLA
GLVAAFFAFRYVLQLQRNPMKALYPLTTKELKTSDPGGVGGEGEEGAEGGGFDEA
KLAEAREMIRYMALVSAMERTEHKARKKGTSALLSSKVTNMVLRKRNKARYSPLHN
EDEAGDEDEL

Amino acids 1-22 corresponding to the N-terminal signal peptide sequence are indicated in italics. 24 amino acids defining the epitope recognized by the antibody Nb1 are indicated in bold letters and underlined:
V101, G116, A117, D280, E281, G287, F289, Y291, H306, T307, S308, Y309, A310, R313, P343, N582, Y637, E648, A651, Y652, S653, H654, Q655 and D660.
**>HSV-1 gB amino acids 31-904 used for primary immunization including a C-terminal Linker/His-tag sequence and a Y889A point mutation indicated in bold (SEQ ID NO: 3) based on UniProt P06437** 
APTSPGTPGVAAATQAANGGPATPAPPPLGAAPTGDPKPKKNKKPKNPTPPRPAG
DNATVAAGHATLREHLRDIKAENTDANFYVCPPPTGATVVQFEQPRRCPTRPEGQ
NYTEGIAVVFKENIAPYKFKATMYYKDVTVSQVWFGHRYSQFMGIFEDRAPVPFEE
VIDKINAKGVCRSTAKYVRNNLETTAFHRDDHETDMELKPANAATRTSRGWHTTDL
KYNPSRVEAFHRYGTTVNCIVEEVDARSVYPYDEFVLATGDFVYMSPFYGYREGSH
TEHTSYAADRFKQVDGFYARDLTTKARATAPTTRNLLTTPKFTVAWDWVPKRPSVC
TMTKWQEVDEMLRSEYGGSFRFSSDAISTTFTTNLTEYPLSRVDLGDCIGKDARDA
MDRIFARRYNATHIKVGQPQYYLANGGFLIAYQPLLSNTLAELYVREHLREQSRKPP
NPTPPPPGASANASVERIKTTSSIEFARLQFTYNHIQRHVNDMLGRVAIAWCELQNH
ELTLWNEARKLNPNAIASVTVGRRVSARMLGDVMAVSTCVPVAADNVIVQNSMRIS
SRPGACYSRPLVSFRYEDQGPLVEGQLGENNELRLTRDAIEPCTVGHRRYFTFGG
GYVYFEEYAYSHQLSRADITTVSTFIDLNITMLEDHEFVPLEVYTRHEIKDSGLLDYTE
VQRRNQLHDLRFADIDTVIHADANAAMFAGLGAFFEGMGDLGRAVGKVVMGIVGG
VVSAVSGVSSFMSNPFGALAVGLLVLAGLAAAFFAFRYVMRLQSNPMKALYPLTTK
ELKNPTNPDASGEGEEGGDFDEAKLAEAREMIRYMALVSAMERTEHKAKKKGTSA
LLSAKVTDMVMRKRRNTNATQVPNKDGDADEDDLQLGSGSGSGSGSHHHHHH
**>HSV-1 gB amino acids 31-904 used for immunogen production - additional boost including a C-terminal Linker/His-tag sequence and H516P and Y889A point mutations indicated in bold (SEQ ID NO: 4) based on UniProt P06437** 
APTSPGTPGVAAATQAANGGPATPAPPPLGAAPTGDPKPKKNKKPKNPTPPRPAG
DNATVAAGHATLREHLRDIKAENTDANFYVCPPPTGATVVQFEQPRRCPTRPEGQ
NYTEGIAVVFKENIAPYKFKATMYYKDVTVSQVWFGHRYSQFMGIFEDRAPVPFEE
VIDKINAKGVCRSTAKYVRNNLETTAFHRDDHETDMELKPANAATRTSRGWHTTDL
KYNPSRVEAFHRYGTTVNCIVEEVDARSVYPYDEFVLATGDFVYMSPFYGYREGSH
TEHTSYAADRFKQVDGFYARDLTTKARATAPTTRNLLTTPKFTVAWDWVPKRPSVC
TMTKWQEVDEMLRSEYGGSFRFSSDAISTTFTTNLTEYPLSRVDLGDCIGKDARDA
 
MDRIFARRYNATHIKVGQPQYYLANGGFLIAYQPLLSNTLAELYVREHLREQSRKPP
NPTPPPPGASANASVERIKTTSSIEFARLQFTYNHIQRPVNDMLGRVAIAWCELQNH
ELTLWNEARKLNPNAIASVTVGRRVSARMLGDVMAVSTCVPVAADNVIVQNSMRIS
SRPGACYSRPLVSFRYEDQGPLVEGQLGENNELRLTRDAIEPCTVGHRRYFTFGG
GYVYFEEYAYSHQLSRADITTVSTFIDLNITMLEDHEFVPLEVYTRHEIKDSGLLDYTE
VQRRNQLHDLRFADIDTVIHADANAAMFAGLGAFFEGMGDLGRAVGKVVMGIVGG
VVSAVSGVSSFMSNPFGALAVGLLVLAGLAAAFFAFRYVMRLQSNPMKALYPLTTK
ELKNPTNPDASGEGEEGGDFDEAKLAEAREMIRYMALVSAMERTEHKAKKKGTSA
LLSAKVTDMVMRKRRNTNATQVPNKDGDADEDDLQLGSGSGSGSGSHHHHHH
**>HSV-1 gB stabilised amino acids 31-904 used for structure determination including a linker, a HRV3C protease site and a Strepll tag sequence (SEQ ID NO: 5) based on UniProt P06437** 
APTSPGTPGVAAATQAANGGPATPAPPPLGAAPTGDPKPKKNKKPKNPTPPRPAG
DNATVAAGHATLREHLRDIKAENTDANFYVCPPPTGATVVQFEQPRRCPTRPEGQ
NYTEGIAVVFKENIAPYKFKATMYYKDVTVSQVWFGHRYSQFMGIFEDRAPVPFEE
VIDKINAKGVCRSTAKYVRNNLETTAFHRDDHETDMELKPANAATRTSRGWHTTDL
KYNPSRVEAFHRYGTTVNCIVEEVDARSVYPYDEFVLATGDFVYMSPFYGYREGSH
TEHTSYAADRFKQVDGFYARDLTTKARATAPTTRNLLTTPKFTVAWDWVPKRPSVC
TMTKWQEVDEMLRSEYGGSFRFSSDAICTTFTTNLTEYPLSRVDLGDCIGKDARDA
MDRIFARRYNATHIKVGQPQYYLANGGFLIAYQPLLSNTLAELYVREHLREQSRKPP
NPTPPPPGASANASVERIKTTSSIEFARLQFTYNHIQRPVNDMLGRVAIAWCELCNH
ELTLWNEARKLNPNAIASVTVGRRVSARMLGDVMAVSTCVPVAADNVIVQNSMRIS
SRPGACYSRPLVSFRYEDQGPLVEGQLGENNELRLTRDAIEPCTVGHRRYFTFGG
GYVYFEEYAYSHQLSRADITTVSTFIDLNITMLEDHEFVPLEVYTRHEIKDSGLLDYTE
VQRRVQLHDLRFADIDTVIHADANAAMFAGLGAFFEGMGDLGRAVGKVVMGIVGG
VVSAVSGVSSFMSNPFGALAVGLLVLAGLAAAFFAFRYVMRLQSNPMKALYPLTTK
ELKNPTNPDASGEGEEGGDFDEAKLAEAREMIRYMALVSAMERTEHKAKKKGTSA
LLSAKVTDMVMRKRRNTNYTQVPNKDGDADEDDLQLGSGSGTLEVLFQGPGGSG
SAWSHPQFEKGGGSGGGSGGSAWSHPQFEK
**> HSV-2 gB amino acids 23-904 used for structure determination including a linker, a HRV3C protease site and a Strepll tag sequence (SEQ ID NO: 6) based on UniProt P06763** 
APAAPAAPRASGGVAATVAANGGPASRPPPVPSPATTKARKRKTKKPPKRPEATP
PPDANATVAAGHATLRAHLREIKVENADAQFYVCPPPTGATVVQFEQPRRCPTRPE
GQNYTEGIAVVFKENIAPYKFKATMYYKDVTVSQVWFGHRYSQFMGIFEDRAPVPF
EEVIDKINAKGVCRSTAKYVRNNMETTAFHRDDHETDMELKPAKVATRTSRGWHTT
DLKYNPSRVEAFHRYGTTVNCIVEEVDARSVYPYDEFVLATGDFVYMSPFYGYREG
SHTEHTSYAADRFKQVDGFYARDLTTKARATSPTTRNLLTTPKFTVAWDWVPKRPA
VCTMTKWQEVDEMLRAEYGGSFRFSSDAISTTFTTNLTQYSLSRVDLGDCIGRDAR
EAIDRMFARKYNATHIKVGQPQYYLATGGFLIAYQPLLSNTLAELYVREYMREQDRK
PRNATPAPLREAPSANASVERIKTTSSIEFARLQFTYNHIQRHVNDMLGRIAVAWCE
LQNHELTLWNEARKLNPNAIASATVGRRVSARMLGDVMAVSTCVPVAPDNVIVQNS
MRVSSRPGTCYSRPLVSFRYEDQGPLIEGQLGENNELRLTRDALEPCTVGHRRYFI
FGGGYVYFEEYAYSHQLSRADVTTVSTFIDLNITMLEDHEFVPLEVYTRHEIKDSGLL
DYTEVQRRNQLHDLRFADIDTVIRADANAAMFAGLCAFFEGMGDLGRAVGKVVMG
 
VVGGVVSAVSGVSSFMSNPFGALAVGLLVLAGLVAAFFAFRYVLQLQRNPMKALYP
LTTKELKTSDPGGVGGEGEEGAEGGGFDEAKLAEAREMIRYMALVSAMERTEHKA
RKKGTSALLSSKVTNMVLRKRNKARYSPLHNEDEAGDEDELGTLEVLFQGPGGSG
SAWSHPQFEKGGGSGGGSGGSAWSHPQFEK
**>HSV-1 gB-sfEGFP gB amino acids 31-904 and GFP amino acids 2-238 used as transfected GFP-fusion (SEQ ID NO: 7) based on UniProt P06437 and GenBank MH644049.1** 
APTSPGTPGVAAATQAANGGPATPAPPALGAAPTGDPKPKKNKKPKNPTPPRPAG
DNATVAAGHATLREHLRDIKAENTDANFYVCPPPTGATVVQFEQPRRCPTRPEGQ
NYTEGIAVVFKENIAPYKFKATMYYKDVTVSQVWFGHRYSQFMGIFEDRAPVPFEE
VIDKINAKGVCRSTAKYVRNNLETTAFHRDDHETDMELKPANAATRTSRGWHTTDL
KYNPSRVEAFHRYGTTVNCIVEEVDARSVYPYDEFVLATGDFVYMSPFYGYREGSH
TEHTSYAADRFKQVDGFYARDLTTKARATAPTTRNLLTTPKFTVAWDWVPKRPSVC
TMTKWQEVDEMLRSEYGGSFRFSSDAISTTFTTNLTEYPLSRVDLGDCIGKDARDA
MDRIFARRYNATHIKVGQPQYYLANGGFLIAYQPLLSNTLAELYVREHLREQSRKPP
NPTPPPPGASANASVERIKTTSSIEFARLQFTYNHIQRHVNDMLGRVAIAWCELQNH
ELTLWNEARKLNPNAIASVTVGRRVSARMLGDVMAVSTCVPVAADNVIVQNSMRIS
SRPGACYSRPLVSFRYEDQGPLVEGQLGENNELRLTRDAIEPCTVGHRRYFTFGG
GYVYFEEYAYSHQLSRADITTVSTFIDLNITMLEDHEFVPLEVYTRHEIKDSGLLDYTE
VQRRNQLHDLRFADIDTVIHADANAAMFAGLGAFFEGMGDLGRAVGKVVMGIVGG
VVSAVSGVSSFMSNPFGALAVGLLVLAGLAAAFFAFRYVMRLQSNPMKALYPLTTK
ELKNPTNPDASGEGEEGGDFDEAKLAEAREMIRYMALVSAMERTEHKAKKKGTSA
LLSAKVTDMVMRKRRNTNYTQVPNKDGDADEDDLGTGSSGVSKGEELFTGVVPIL
VELDGDVNGHKFSVRGEGEGDATNGKLTLKFICTTGKLPVPWPTLVTTLTYGVQCF
SRYPDHMKQHDFFKSAMPEGYVQERTISFKDDGTYKTRAEVKFEGDTLVNRIELKG
IDFKEDGNILGHKLEYNFNSHNVYITADKQKNGIKANFKIRHNVEDGSVQLADHYQQ
NTPIGDGPVLLPDNHYLSTQSKLSKDPNEKRDHMVLLEFVTAAGITLGMDELYK
**>HSV-2 gB-sfEGFP gB amino acids 23-904 and GFP amino acids 2-238 used as transfected GFP-fusion (SEQ ID NO: 8) based on UniProt P06763 and GenBank MH644049.1** 
APAAPAAPRASGGVAATVAANGGPASRPPPVPSPATTKARKRKTKKPPKRPEATP
PPDANATVAAGHATLRAHLREIKVENADAQFYVCPPPTGATVVQFEQPRRCPTRPE
GQNYTEGIAVVFKENIAPYKFKATMYYKDVTVSQVWFGHRYSQFMGIFEDRAPVPF
EEVIDKINAKGVCRSTAKYVRNNMETTAFHRDDHETDMELKPAKVATRTSRGWHTT
DLKYNPSRVEAFHRYGTTVNCIVEEVDARSVYPYDEFVLATGDFVYMSPFYGYREG
SHTEHTSYAADRFKQVDGFYARDLTTKARATSPTTRNLLTTPKFTVAWDWVPKRPA
VCTMTKWQEVDEMLRAEYGGSFRFSSDAISTTFTTNLTQYSLSRVDLGDCIGRDAR
EAIDRMFARKYNATHIKVGQPQYYLATGGFLIAYQPLLSNTLAELYVREYMREQDRK
PRNATPAPLREAPSANASVERIKTTSSIEFARLQFTYNHIQRHVNDMLGRIAVAWCE
LQNHELTLWNEARKLNPNAIASATVGRRVSARMLGDVMAVSTCVPVAPDNVIVQNS
MRVSSRPGTCYSRPLVSFRYEDQGPLIEGQLGENNELRLTRDALEPCTVGHRRYFI
FGGGYVYFEEYAYSHQLSRADVTTVSTFIDLNITMLEDHEFVPLEVYTRHEIKDSGLL
DYTEVQRRNQLHDLRFADIDTVIRADANAAMFAGLCAFFEGMGDLGRAVGKVVMG
VVGGVVSAVSGVSSFMSNPFGALAVGLLVLAGLVAAFFAFRYVLQLQRNPMKALYP
LTTKELKTSDPGGVGGEGEEGAEGGGFDEAKLAEAREMIRYMALVSAMERTEHKA
 
RKKGTSALLSSKVTNMVLRKRNKARYSPLHNEDEAGDEDELGTGSSGVSKGEELF
TGVVPILVELDGDVNGHKFSVRGEGEGDATNGKLTLKFICTTGKLPVPWPTLVTTLT
YGVQCFSRYPDHMKQHDFFKSAMPEGYVQERTISFKDDGTYKTRAEVKFEGDTLV
NRIELKGIDFKEDGNILGHKLEYNFNSHNVYITADKQKNGIKANFKIRHNVEDGSVQL
ADHYQQNTPIGDGPVLLPDNHYLSTQSKLSKDPNEKRDHMVLLEFVTAAGITLGMD
ELYK
**>VZV gB-sfEGFP gB amino acids 72-931 and GFP amino acids 2-238 used as transfected GFP-fusion (SEQ ID NO: 9) based on UniProt Q4JR05 and GenBank MH644049.1** 
VSPSSFYESLQVEPTQSEDITRSAHLGDGDEIREAIHKSQDAETKPTFYVCPPPTGS
TIVRLEPPRTCPDYHLGKNFTEGIAVVYKENIAAYKFKATVYYKDVIVSTAWAGSSYT
QITNRYADRVPIPVSEITDTIDKFGKCSSKATYVRNNHKVEAFNEDKNPQDMPLIASK
YNSVGSKAWHTTNDTYMVAGTPGTYRTGTSVNCIIEEVEARSIFPYDSFGLSTGDIIY
MSPFFGLRDGAYREHSNYAMDRFHQFEGYRQRDLDTRALLEPAARNFLVTPHLTV
GWNWKPKRTEVCSLVKWREVEDVVRDEYAHNFRFTMKTLSTTFISETNEFNLNQIH
LSQCVKEEARAIINRIYTTRYNSSHVRTGDIQTYLARGGFVVVFQPLLSNSLARLYLQ
ELVRENTNHSPQKHPTRNTRSRRSVPVELRANRTITTTSSVEFAMLQFTYDHIQEHV
NEMLARISSSWCQLQNRERALWSGLFPINPSALASTILDQRVKARILGDVISVSNCP
ELGSDTRIILQNSMRVSGSTTRCYSRPLISIVSLNGSGTVEGQLGTDNELIMSRDLLE
PCVANHKRYFLFGHHYVYYEDYRYVREIAVHDVGMISTYVDLNLTLLKDREFMPLQV
YTRDELRDTGLLDYSEIQRRNQMHSLRFYDIDKVVQYDSGTAIMQGMAQFFQGLGT
AGQAVGHVVLGATGALLSTVHGFTTFLSNPFGALAVGLLVLAGLVAAFFAYRYVLKL
KTSPMKALYPLTTKGLKQLPEGMDPFAEKPNATDTPIEEIGDSQNTEPSVNSGFDPD
KFREAQEMIKYMTLVSAAERQESKARKKNKTSALLTSRLTGLALRNRRGYSRVRTE
NVTGVSGTGSSGVSKGEELFTGVVPILVELDGDVNGHKFSVRGEGEGDATNGKLTL
KFICTTGKLPVPWPTLVTTLTYGVQCFSRYPDHMKQHDFFKSAMPEGYVQERTISF
KDDGTYKTRAEVKFEGDTLVNRIELKGIDFKEDGNILGHKLEYNFNSHNVYITADKQK
NGIKANFKIRHNVEDGSVQLADHYQQNTPIGDGPVLLPDNHYLSTQSKLSKDPNEK
RDHMVLLEFVTAAGITLGMDELYK
**>HCMV gB-sfEGFP gB amino acids 25-907 and GFP amino acids 2-238 used as transfected GFP-fusion (SEQ ID NO: 10) based on UniProt F5HB53 and GenBank MH644049.1** 
SSSTRGTSATHSHHSSHTTSAAHSRSGSVSQRVTSSQTVSHGVNETIYNTTLKYGD
VVGVNTTKYPYRVCSMAQGTDLIRFERNIVCTSMKPINEDLDEGIMVVYKRNIVAHT
FKVRVYQKVLTFRRSYAYIHTTYLLGSNTEYVAPPMWEIHHINSHSQCYSSYSRVIA
GTVFVAYHRDSYENKTMQLMPDDYSNTHSTRYVTVKDQWHSRGSTWLYRETCNL
NCMVTITTARSKYPYHFFATSTGDVVDISPFYNGTNRNASYFGENADKFFIFPNYTIV
SDFGRPNSALETHRLVAFLERADSVISWDIQDEKNVTCQLTFWEASERTIRSEAEDS
YHFSSAKMTATFLSKKQEVNMSDSALDCVRDEAINKLQQIFNTSYNQTYEKYGNVS
VFETTGGLVVFWQGIKQKSLVELERLANRSSLNLTHNRTKRSTDGNNATHLSNMES
VHNLVYAQLQFTYDTLRGYINRALAQIAEAWCVDQRRTLEVFKELSKINPSAILSAIY
NKPIAARFMGDVLGLASCVTINQTSVKVLRDMNVKESPGRCYSRPVVIFNFANSSYV
QYGQLGEDNEILLGNHRTEECQLPSLKIFIAGNSAYEYVDYLFKRMIDLSSISTVDSMI
ALDIDPLENTDFRVLELYSQKELRSSNVFDLEEIMREFNSYKQRVKYVEDKVVDPLP
PYLKGLDDLMSGLGAAGKAVGVAIGAVGGAVASVVEGVATFLKNPFGAFTIILVAIAV
VIITYLIYTRQRRLCTQPLQNLFPYLVSADGTTVTSGSTKDTSLQAPPSYEESVYNSG
 
RKGPGPPSSDASTAAPPYTNEQAYQMLLALARLDAEQRAQQNGTDSLDGRTGTQ
DKGQKPNLLDRLRHRKNGYRHLKDSDEEENVGTGSSGVSKGEELFTGVVPILVELD
GDVNGHKFSVRGEGEGDATNGKLTLKFICTTGKLPVPWPTLVTTLTYGVQCFSRYP
DHMKQHDFFKSAMPEGYVQERTISFKDDGTYKTRAEVKFEGDTLVNRIELKGIDFKE
DGNILGHKLEYNFNSHNVYITADKQKNGIKANFKIRHNVEDGSVQLADHYQQNTPIG
DGPVLLPDNHYLSTQSKLSKDPNEKRDHMVLLEFVTAAGITLGMDELYK
**>EBV gB-sfEGFP gB amino acids 22-857 and GFP amino acids 2-238 used as transfected GFP-fusion (SEQ ID NO: 11) based on UniProt P03188 and GenBank MH644049.1** 
AQTPEQPAPPATTVQPTATRQQTSFPFRVCELSSHGDLFRFSSDIQCPSFGTRENH
TEGLLMVFKDNIIPYSFKVRSYTKIVTNILIYNGWYADSVTNRHEEKFSVDSYETDQM
DTIYQCYNAVKMTKDGLTRVYVDRDGVNITVNLKPTGGLANGVRRYASQTELYDAP
GWLIWTYRTRTTVNCLITDMMAKSNSPFDFFVTTTGQTVEMSPFYDGKNKETFHER
ADSFHVRTNYKIVDYDNRGTNPQGERRAFLDKGTYTLSWKLENRTAYCPLQHWQT
FDSTIATETGKSIHFVTDEGTSSFVTNTTVGIELPDAFKCIEEQVNKTMHEKYEAVQD
RYTKGQEAITYFITSGGLLLAWLPLTPRSLATVKNLTELTTPTSSPPSSPSPPAPSAA
RGSTPAAVLRRRRRDAGNATTPVPPTAPGKSLGTLNNPATVQIQFAYDSLRRQINR
MLGDLARAWCLEQKRQNMVLRELTKINPTTVMSSIYGKAVAAKRLGDVISVSQCVP
VNQATVTLRKSMRVPGSETMCYSRPLVSFSFINDTKTYEGQLGTDNEIFLTKKMTEV
CQATSQYYFQSGNEIHVYNDYHHFKTIELDGIATLQTFISLNTSLIENIDFASLELYSR
DEQRASNVFDLEGIFREYNFQAQNIAGLRKDLDNAVSNGRNQFVDGLGELMDSLG
SVGQSITNLVSTVGGLFSSLVSGFISFFKNPFGGMLILVLVAGVVILVISLTRRTRQMS
QQPVQMLYPGIDELAQQHASGEGPGINPISKTELQAIMLALHEQNQEQKRAAQRAA
GPSVASRALQAARDRFPGLRRRRYHDPETAAALLGEAETEFGTGSSGVSKGEELF
TGVVPILVELDGDVNGHKFSVRGEGEGDATNGKLTLKFICTTGKLPVPWPTLVTTLT
YGVQCFSRYPDHMKQHDFFKSAMPEGYVQERTISFKDDGTYKTRAEVKFEGDTLV
NRIELKGIDFKEDGNILGHKLEYNFNSHNVYITADKQKNGIKANFKIRHNVEDGSVQL
ADHYQQNTPIGDGPVLLPDNHYLSTQSKLSKDPNEKRDHMVLLEFVTAAGITLGMD
ELYK
**>VSV G-sfEGFP gB amino acids 17-511 and GFP amino acids 2-238 used as transfected GFP-fusion (SEQ ID NO: 12) based on UniProt B7UCZ5 and GenBank MH644049.1**

KFTIVFPHNQKGNWKNVPSNYHYCPSSSDLNWHNDLIGTALQVKMPKSHKAIQADG
WMCHASKWVTTCDFRWYGPKYITHSIRSFTPSVEQCKESIEQTKQGTWLNPGFPP
QSCGYATVTDAEAVIVQVTPHHVLVDEYTGEWVDSQFINGKCSNYICPTVHNSTTW
HSDYKVKGLCDSNLISMDITFFSEDGELSSLGKEGTGFRSNYFAYETGGKACKMQY
CKHWGVRLPSGVWFEMADKDLFAAARFPECPEGSSISAPSQTSVDVSLIQDVERIL
DYSLCQETWSKIRAGLPISPVDLSYLAPKNPGTGPAFTIINGTLKYFETRYIRVDIAAPI
LSRMVGMISGTTTERELWDDWAPYEDVEIGPNGVLRTSSGYKFPLYMIGHGMLDS
DLHLSSKAQVFEHPHIQDAASQLPDDESLFFGDTGLSKNPIELVEGWFSSWKSSIAS
FFFIIGLIIGLFLVLRVGIHLCIKLKHTKKRQIYTDIEMNRLGKQLGSSVSKGEELFTGVV
PILVELDGDVNGHKFSVRGEGEGDATNGKLTLKFICTTGKLPVPWPTLVTTLTYGVQ
CFSRYPDHMKQHDFFKSAMPEGYVQERTISFKDDGTYKTRAEVKFEGDTLVNRIEL

KGIDFKEDGNILGHKLEYNFNSHNVYITADKQKNGIKANFKIRHNVEDGSVQLADHY
QQNTPIGDGPVLLPDNHYLSTQSKLSKDPNEKRDHMVLLEFVTAAGITLGMDELYK

### Sequences of VHH antibodies

> Nb1_gbHSV (1-119) VHH sequence (SEQ ID NO:13)
QVQLVESGGGSVQPGGSLRLSCAASASGRLSMSGALGWYRQVQGKSRELVATIT
DRSSTNYADSVKGRFTISIDDAENTMYLQMNSLKPEDTGVYYCNARWRGMNVWG
KGTRVTVSS

The CDR1, CDR2 and CDR3 sequences are indicated in bold letters.

**Nb1_gbHSV** paratope list is as follows: Q1, A26, S27, G28, R29, M32, G34, A35, Y39, L49, T52, T54, D55, R56, S58, T59, N60, Y61, K66, R100, W101, R102, G103, M104.
**>Nb1_gbHSV_CDR1 (26-37) (SEQ ID NO: 14)**
   ASGRLSMSGALG
**>Nb1_gbHSV_CDR2 (52-59) (SEQ ID NO: 15)**
   TITDRSST
**>Nb1_gbHSV_CDR3 (98-106) (SEQ ID NO: 16)**
   NARWRGMNV
> **Nb2_gbHSV (1-117) VHH sequence (SEQ ID NO: 17)**

QVQLVESGGGLVQPGGSLRLSCAASGVTFSSSVMSWHRQAPGQQREFVASITSA
GVTDYADSVKGRATISRDNAQNTVYLQMNSLKPEDTAVYYCSAVPRPYQLQYWGQ
GTRVTVSS

The CDR1, CDR2 and CDR3 sequences are indicated in bold letters.
**>Nb2_gbHSV_CDR1 (26-37) (SEQ ID NO: 18)**
   ASGVTFSSSVMS
**>Nb2_gbHSV_CDR2 (52-59) (SEQ ID NO: 19)**
   SITSAGVTD
**>Nb2_gbHSV_CDR3 (96-106) (SEQ ID NO: 20)**
   SAVPRPYQLQY

### Examples

The present invention is further described in more detail by the following examples which are only provided for illustrating the invention and which are not to be construed as limiting the scope of the invention. The following material and methods were used in the Examples.

### Example 1: Generation of an HSV-1 gB pre-fusion immunogen

The recombinant full-length gB protein, embedded in extracellular vesicles was used to generate and select specific nanobodies directed against this protein. Overexpression of gB in BHK21 cells results in the formation of extracellular vesicles (Zeev-Ben-Mordehai *et al.*, 2014), studded with the protein on the surface in pre- and post-fusion conformation. Purification of gB vesicles was performed similar to the method described in (Zeev-Ben-Mordehai *et al.*, 2014). 30x T175 flasks of BHK21 cells were transfected using Lipofectamine 2000 with a plasmid coding for HSV-1 gB with the point mutation Y889A (SEQ ID NO: 3) to increase formation of extracellular vesicles. 48h and 72h after transfection, cell supernatants were collected and concentrated using a Vivaflow (Sartorius) diafiltration system with a 100 kDa MW Cut-off. Concentrated vesicles in the remaining supernatant were concentrated by ultracentrifugation at 100 000 x g for 2 h at 4°C through a 20% sucrose cushion. Vesicle pellets were rehydrated over night at 4°C in ~100 µL PBS containing 250 mM sorbitol / T175 flask start material. Vesicles were analysed by SDS PAGE in comparison with BSA standard to estimate the protein amount.

Expression and purification of the gB protein yielded enough protein for immunisations. The total amount of gB protein in vesicles from the supernatant collected 48 h after transfection was ~3 mg as estimated from the band size and intensity in comparison to the BSA standard as shown in Figure 1.

For the last injection vesicles were used containing HSV-1 gB locked in its prefusion conformation in order to focus the generation of nanobodies directed against this conformation. Vesicles were produced as described before, but using a construct containing the additional mutation H516P (SEQ ID NO: 4) which prevents gB to transition into the postfusion conformation (Vollmer *et al*, 2020) using 25 x T175 flasks of BHK21. The final amount of gB in vesicles was determined in comparison with BSA standard to be ~2 mg.

### Example 2: Production of Nb1_gbHSV in E. coli

Nb1_gbHSV was produced by cytoplasmic expression in *E.coli*, using NEB Shuffle (NEB #C3028J) as an expression host, which yields a nanobody with a partially formed disulfide bond. In brief, bacteria were transformed with a plasmid encoding a His₁₄-NEDD8-VHH fusion under control of a T5-lac promoter (Pleiner *et al.*, 2015). A single glycine (Gly, G) was included between NEDD8 and the nanobody. This was necessary to allow subsequent tag-cleavage. Expression was at 25°C in TB medium, induction with 100 µM IPTG for 5 hours. Cells were pelleted, resuspended in 50 mM Tris/HCl pH 7.5, 300 mM NaCl, lysed by a freeze-thawed cycle and sonication. The lysate was cleared by ultracentrifugation and applied to a Ni²⁺ chelate matrix. After extensive washing, the Ni(II)-bound VHH was eluted by cleaving the N-terminal His₁₄-NEDD8 tag with bdNEDP1 protease (Frey & Gorlich, 2014a, b). Subsequently, the formation of the structural disulfide bond was completed as described in WO 2024/256467.

### Example 3: Alpaca immunisation elicits neutralizing VHH antibody

Purified VHH antibodies were tested for neutralization of HSV-1. Infectious HSV-1 particles were incubated with different concentrations of two different VHH antibodies before cell layer infection. Plaques were counted and numbers were compared to plaque numbers observed in controls where HSV-1 was incubated in buffer. By this, neutralizing activity of the different VHH antibodies in different concentrations is determined.

Test of the two different VHH antibodies revealed only Nb1_gbHSV to have a neutralizing function for the complete range of tested concentrations (Figure 2a). Other nanobodies like Nb2_gbHSV show no neutralizing activity when compared to the buffer control.

### Example 4: Nb1_gbHSV prevents HSV-1 infection

The complex structure suggests that interaction of the VHH antibody with the gB protein in pre-fusion conformation prevents the protein to perform membrane fusion as domains I and IV are crosslinked, therefore preventing the necessary conformational changes essential to bind and pull the host cell membrane towards the viral membrane. This makes the VHH antibody a potential inhibitor of HSV-1 infection.

To test for neutralization of the VHH antibody, plaque reduction assays were performed. Vero cells were seeded in 24 well plates and grown over night to form a confluent cell layer. Prior to cell layer infection using ~20 pfu/well, viruses were pre-incubated for 30 min at room temperature (RT) with different concentrations of VHH antibodies. Subsequently infection was performed for 1.5 h at RT before inoculum was removed and cells were washed with PBS. After buffer removal cells were overlaid with 1.2% Avicell in DMEM and 5% FBS to prevent secondary infection via the cell media and to promote cell to cell spread (plaque formation). 48 - 72h after infection cells were fixed with 4% paraformaldehyde in H₂O and stained with 0.1% crystal violet in 2% Ethanol. Plaques were manually counted. The IC₅₀ values were calculated using the AAT Bioquest calculator (https://www.aatbio.com/tools/ic50-calculator).

Using the plaque reduction assay, the IC₅₀ value for Nb1_gbHSV was determined and is shown in Figure 2b. For this HSV-1 was preincubated with VHH antibody concentrations ranging from 0.01 nM - 10 µM. Experiments were performed in biological triplicates while using 3 wells per VHH antibody concentration each time. The determined IC₅₀ value for Nb1_gbHSV is 1.2 nM showing the high neutralization potential of Nb1_gbHSV, even in low concentrations (Figure 2b) and confirming the neutralization activity seen in the previous analysis (Figure 2a).

### Example 5: Nb1_gbHSV shows cross-reactivity

ARPE-19 cells were grown in Ibidi µ-Slide 8 Well chambered coverslips for 24 h before transient transfection using Lipofectamine 2000. Cells were transfected with plasmids for expression of different gB homologues, namely HSV-1, HSV-2, VZV, HCMV or EBV. In addition, as negative control the structurally related glycoprotein G from Vesicular Stomatitis Virus was transfected. All constructs are based on the respective wild type sequence, while encoding a C-terminally added fluorescent sfEGFP tag. 1 µg DNA + 1 µL Lipofectamine / well in 200 µL DMEM media were used for transfection. 48 h post transfection cells were washed with PBS before addition of 200 µL fresh DMEM media including 1 µM Nb1_gbHSV nanobody labelled through a C-terminal ectopic cysteine by Alexa647-maleimide (Pleiner *et al.*, 2015). After 1 h incubation at 37°C, cells were washed with PBS and the media was replaced. Cells were imaged live with constant settings. Images were analysed using the FIJI software and the JaCoP plugin (Bolte & Cordelieres, 2006) to calculate the Pearson correlation coefficients of the measured fluorescent signal in the green and red channel.

For all transfected proteins, fluorescent sfEGFP signal can be found in cells. Cells expressing either the HSV-1 or HSV-2 gB protein are recognized by the fluorescently labelled nanobody, seen by colocalization of both signals. After imaging the cells, Pearson correlation coefficients of the measured fluorescent signal in the green and red channel were calculated and are shown as box-and-whisker plot (Figure 3b). The median value is marked by a horizontal line, while the box marks the limits of the upper and lower quartile value and the whiskers showing the min and max value. Outliers are marked as points outside the whisker range. Example fluorescent images of gB transfected cell stained with the Alexa647 labelled nanobody show the colocalization of the fluorescent signals in cells transfected with HSV-1 and HSV-2 gB while EBV gB transfected cells show only fluorescent signal in the green (GFP) channel (Figure 3a).

### Example 6: Nb1_gbHSV is hyperthermostable

Nb1_gbHSV was produced as described in Example 3 but using the E.coli strain NEB Express (NEB #C30371) as an expression host, yielding a VHH antibody whose two structural cysteines remained reduced. To form the structural disulfide bond, the inventors followed the strategy described in WO 2024/256467 (PCT/EP2024/066244), i.e., they incubated 50 µM of the nanobody with 2 mM oxidized glutathione (GSSG) and 1.5 µM each of the *E.coli* protein disulfide isomerases DsbA, DsbC, and DsbG at 37°C for three hours.

The reduced and disulfide-bonded forms were then subjected to Differential Scanning Fluorimetry (DSF), which exploits that thermal unfolding exposes aromatic/hydrophobic residues, which then bind and enhance the fluorescence of the added SYPRO Orange dye. Assays were performed in a volume of 20 µl, at 1 mg/ml VHH concentration in 50 mM Tris/HCl, 150 mM NaCl (pH 8.0 at 20°C), and 1 x dye (diluted from a 5000x stock; Life Technologies). Two sample replicates were pipetted into a Hard-Shell^{®} 96-well plate (Bio-Rad). The plate was sealed with transparent MicroSeal^{®} 'B' Seal (Bio-Rad), briefly centrifuged to remove air bubbles, and placed in a CFX96 Real-Time System (C1000 Thermal Cycler, BioRad). Samples were incubated for 5 min at 20°C and then heated in 1°C increments of 45 seconds up to 95°C. Fluorescence was measured at the end of each step with 532 nm excitation and a 555 nm long pass filter. Melting temperatures are defined as the inflection point of the first melting peak. Note that the reduced Nb1_gbHSV showed a clear melting signal with a Tm of 55°C, while the disulfide-bond VHH did not unfold and remained stable throughout the entire tested temperature range. Data are shown in Figure 4.

### Example 7: The Nb1_gbHSV epitope on pre-fusion gB is determined by cryo-electron microscopy (cryoEM)

Mutationally prefusion stabilised gB protein was incubated with Nb1_gbHSV for 10 min on ice. The mixture was then used for cryoEM grid preparation as described in (Niebling *et al*, 2022). 3.5 µL of the mixture were added to glow discharged Quantifoil R2.2 copper grids before a 3 sec blotting step followed by plunging into a liquid ethane/propane mixture using a Thermo Fisher Scientific (TFS) Vitrobot Mark IV. Data acquisition of 3,066 movies was performed as described in (Williams *et al*, 2023) using a pixel size of 0.83 Å/px and a total accumulated electron dose of 44.9 e⁻/Å² with 1 e⁻/Å² per frame. All processing steps were performed in CryoSPARC (Punjani *et al*, 2017). H-bonds and interacting residues were determined using ChimeraX (Meng *et al*., 2023) and PDBe PISA (Krissinel & Henrick, 2007).

The structure of the gB proteins of HSV2 (Figure 5) and HSV1 (Figure 6) in complex with the Nb1_gbHSV nanobody were determined to a resolution of ~3 Å. Interacting regions were of high enough resolution to confidently build the protein maps in these regions. The final structure for HSV-1 gB shows a total of 84 contacts including 17 H-bonds and 8 salt bridges as determined by ChimeraX (Table 1). For HSV-2 gB the final structure shows 111 contacts including 14 H-bonds and 6 salt bridges (Table 1). This analysis defines the epitope as well as the paratope on both proteins (Figure 6 - 8, Table 1).

Interacting residues include for HSV-1 gB: A106, N108, G121, A122, D285, E286, G292, F294, Y296, H311, T312, S313, Y314, A315, D317, R318, P348, K349, N585, Y640, Y649, E651, A654, Y655, S656, H657, Q658, L659, D663 and Nb1_gbHSV: Q1, A26, S27, G28, R29, M32, G34, A35, Y39, L49, T52, T54, D55, R56, S58, T59, N60, Y61, K66, R100, W101, R102, G103, M104.

Interacting residues include for HSV2 gB: V101, G116, A117, D280, E281, G287, F289, Y291, H306, T307, S308, Y309, A310, R313, P343, N582, Y637, Y646, E648, E649, A651, Y652, S653, H654, Q655, D660 and Nb1_gbHSV: Q1, A26, S27, G28, R29, M32, G34, A35, Y39, L49, T52, T54, D55, R56, S58, T59, N60, Y61, R100, W101, R102, G103, M104.

gB is a homotrimeric complex. The structure shows that for each gB trimer, three nanobodies are able to bind without steric hinderance. The structural analysis of the epitope shows that it is distributed over three different domains, namely domain I and III of one protomer, as well as domain IV of the adjacent protomer. This binding mechanism suggests that the interaction of the nanobody restricts gB in its prefusion form and thereby prevents the conformational changes necessary for the membrane fusion process during infection from pre- to postfusion (Figure 9).

### Example 8: Nb1_gbHSV has a high affinity to prefusion gB

The affinity of Nb1_gbHSV to the prefusion and postfusion conformation of gB was determined by grating-coupled interferometry (GCI) on a Creoptix WAVEdelta instrument (Creoptix AG, Wädenswil, Switzeland). This device allows label-free and highly sensitive determination of binding kinetics using the waveRAPID mode where repeated analyte pulses of increasing duration onto the WAVEchip are used to simulate injections of increasing analyte concentrations. Therefore, kinetics measurements are fast and require less sample compared to traditional affinity measurements (Kartal *et al*, 2021).

For this, either pre-fusion stabilised gB or a wild type ectodomain construct that adopts the post-fusion conformation was immobilised on quasi-planar polycarboxylate polymer (PCP) WAVEchips with four channels (Creoptix AG). The chips were coated with Strep-Tactin XT via amine coupling using the TwinStrep-tag capturing kit from IBA Lifesciences. For the kinetics measurements prefusion-stabilised gB or postfusion gB (ectodomain of wildtype) were immobilised on separate channels on the WAVEchip via a C-terminal TwinStrep-tag to a density of 400 pg/mm². During gB immobilisation and kinetics measurements 20 mM HEPES, pH 8.0, 300 mM NaCl was used as running buffer. Kinetics measurements were performed using a concentration of 200 nM Nb1_gbHSV while 1 µM of a minimal binding construct of the gB receptor PILR*α* (aa 26-154) was used as control. All measurements were performed at 25°C and the data was evaluated using the WAVEcontrol software. All measurements were double referenced against the reference channel (Strep-Tactin XT coated) and a blank. Due to the high affinity of Nb1_gbHSV, the chip was regenerated using 3 x 90 s injections of 3 M GuHCl (IBA Lifesciences) and new pre- and post-fusion gB was immobilised in respective channels as described above before the next kinetics measurement. Tight binder measurements (Nb1_gbHSV) were analysed using traditional fitting and an end crop was set to 1000 sec while for intermediate binder measurements (PILR*α*), the end crop was kept at default. Each measurement was performed three times and the determined KD values were used to calculate the average and standard deviation (Figure 10).

The IgV domain of PILR*α* , which was used as a positive control, showed binding to both pre- and post-fusion gB with KD = 2.9 µM and 1.9 µM, respectively (Figure 10a). The affinity of Nb1_gbHSV for prefusion gB was determined to be KD = 94 pM (standard deviation = 81 pM), while no affinity could be determined for postfusion gB (Figure 10b). The conformational change of gB into post-fusion conformation restricts part of the bound epitope, resulting in the significant reduction in affinity.

### References

Whitley (1996) In: Medical Microbiology, Baron S. (ed.)Galveston (TX)
Bodilsen J, Storgaard M, Larsen L, Wiese L, Helweg-Larsen J, Lebech AM, Brandt C, Ostergaard C, Nielsen H, group Ds (2018) Infectious meningitis and encephalitis in adults in Denmark: a prospective nationwide observational cohort study (DASGIB). Clin Microbiol Infect 24: 1102 e1101-1102 e1105
Bolte S, Cordelieres FP (2006) A guided tour into subcellular colocalization analysis in light microscopy. J Microsc 224: 213-232
Bournazos S, Ravetch JV (2017) Diversification of IgG effector functions. Int Immunol 29: 303-310
Davis-Poynter N, Bell S, Minson T, Browne H (1994) Analysis of the contributions of herpes simplex virus type 1 membrane proteins to the induction of cell-cell fusion. J Virol 68: 7586-7590
Frey S, Görlich D (2014a) A new set of highly efficient, tag-cleaving proteases for purifying recombinant proteins. J Chromatogr A 1337: 95-105
Frey S, Görlich D (2014b) Purification of protein complexes of defined subunit stoichiometry using a set of orthogonal, tag-cleaving proteases. J Chromatogr A 1337: 106-115
Gohring K, Mikeler E, Jahn G, Hamprecht K (2006) Rapid simultaneous detection by real-time PCR of cytomegalovirus UL97 mutations in codons 460 and 520 conferring ganciclovir resistance. J Clin Microbiol 44: 4541-4544
Guttler T, Aksu M, Dickmanns A, Stegmann KM, Gregor K, Rees R, Taxer W, Rymarenko O, Schunemann J, Dienemann C et al (2021) Neutralization of SARS-CoV-2 by highly potent, hyperthermostable, and mutation-tolerant nanobodies. EMBO J 40: e107985
Hamers-Casterman C, Atarhouch T, Muyldermans S, Robinson G, Hamers C, Songa EB, Bendahman N, Hamers R (1993) Naturally occurring antibodies devoid of light chains. Nature 363: 446-448
Heldwein EE (2016) gH/gL supercomplexes at early stages of herpesvirus entry. Curr Opin Virol 18: 1-8
Heldwein EE, Lou H, Bender FC, Cohen GH, Eisenberg RJ, Harrison SC (2006) Crystal structure of glycoprotein B from herpes simplex virus 1. Science 313: 217-220
Herold BC, Visalli RJ, Susmarski N, Brandt CR, Spear PG (1994) Glycoprotein C-independent binding of herpes simplex virus to cells requires cell surface heparan sulphate and glycoprotein B. J Gen Virol 75 ( Pt 6): 1211-1222
Herold BC, WuDunn D, Soltys N, Spear PG (1991) Glycoprotein C of herpes simplex virus type 1 plays a principal role in the adsorption of virus to cells and in infectivity. J Virol 65: 1090-1098
James C, Harfouche M, Welton NJ, Turner KM, Abu-Raddad LJ, Gottlieb SL, Looker KJ (2020) Herpes simplex virus: global infection prevalence and incidence estimates, 2016. Bull World Health Organ 98: 315-329
Kartal O, Andres F, Lai MP, Nehme R, Cottier K (2021) waveRAPID-A Robust Assay for High-Throughput Kinetic Screens with the Creoptix WAVEsystem. SLAS Discov 26: 995-1003
Krissinel E, Henrick K (2007) Inference of macromolecular assemblies from crystalline state. J Mol Biol 372: 774-797
Krummenacher C, Baribaud F, Ponce de Leon M, Baribaud I, Whitbeck JC, Xu R, Cohen GH, Eisenberg RJ (2004) Comparative usage of herpesvirus entry mediator A and nectin-1 by laboratory strains and clinical isolates of herpes simplex virus. Virology 322: 286-299
Levitz RE (1998) Herpes simplex encephalitis: a review. Heart Lung 27: 209-212
Ljungman P (2001) Prophylaxis against herpesvirus infections in transplant recipients. Drugs 61: 187-196
Meng EC, Goddard TD, Pettersen EF, Couch GS, Pearson ZJ, Morris JH, Ferrin TE (2023) UCSF ChimeraX: Tools for structure building and analysis. Protein Sci 32: e4792
Mole L, Ripich S, Margolis D, Holodniy M (1997) The impact of active herpes simplex virus infection on human immunodeficiency virus load. J Infect Dis 176: 766-770
Muyldermans S (2013) Nanobodies: natural single-domain antibodies. Annu Rev Biochem 82: 775-797
Niebling S, Veith K, Vollmer B, Lizarrondo J, Burastero O, Schiller J, Struve Garcia A, Lewe P, Seuring C, Witt S et al (2022) Biophysical Screening Pipeline for Cryo-EM Grid Preparation of Membrane Proteins. Front Mol Biosci 9: 882288
Platt MP, Agalliu D, Cutforth T (2017) Hello from the Other Side: How Autoantibodies Circumvent the Blood-Brain Barrier in Autoimmune Encephalitis. Front Immunol 8: 442
Pleiner T, Bates M, Trakhanov S, Lee CT, Schliep JE, Chug H, Bohning M, Stark H, Urlaub H, Gorlich D (2015) Nanobodies: site-specific labeling for super-resolution imaging, rapid epitope-mapping and native protein complex isolation. Elife 4: e11349
Posavad CM, Koelle DM, Shaughnessy MF, Corey L (1997) Severe genital herpes infections in HIV-infected individuals with impaired herpes simplex virus-specific CD8+ cytotoxic T lymphocyte responses. Proc Natl Acad Sci USA 94: 10289-10294
Punjani A, Rubinstein JL, Fleet DJ, Brubaker MA (2017) cryoSPARC: algorithms for rapid unsupervised cryo-EM structure determination. Nat Methods 14: 290-296
Pyzik M, Kozicky LK, Gandhi AK, Blumberg RS (2023) The therapeutic age of the neonatal Fc receptor. Nat Rev Immunol 23: 415-432
Remeijer L, Maertzdorf J, Doornenbal P, Verjans GM, Osterhaus AD (2001) Herpes simplex virus 1 transmission through corneal transplantation. Lancet 357: 442
Rey FA (2006) Molecular gymnastics at the herpesvirus surface. EMBO Rep 7: 1000-1005
Skouboe MK, Werner M, Mogensen TH (2023) Inborn Errors of Immunity Predisposing to Herpes Simplex Virus Infections of the Central Nervous System. Pathogens 12
Vollmer B, Prazak V, Vasishtan D, Jefferys EE, Hernandez-Duran A, Vallbracht M, Klupp BG, Mettenleiter TC, Backovic M, Rey FA et al (2020) The prefusion structure of herpes simplex virus glycoprotein B. Sci Adv6
Whitley R, Arvin A, Prober C, Burchett S, Corey L, Powell D, Plotkin S, Starr S, Alford C, Connor J et al (1991) A controlled trial comparing vidarabine with acyclovir in neonatal herpes simplex virus infection. Infectious Diseases Collaborative Antiviral Study Group. N Engl J Med 324: 444-449.
Williams HM, Thorkelsson SR, Vogel D, Milewski M, Busch C, Cusack S, Grunewald K, Quemin ERJ, Rosenthal M (2023) Structural insights into viral genome replication by the severe fever with thrombocytopenia syndrome virus L protein. Nucleic Acids Res 51: 1424-1442
Zeev-Ben-Mordehai T, Vasishtan D, Siebert CA, Whittle C, Grunewald K (2014) Extracellular vesicles: a platform for the structure determination of membrane proteins by Cryo-EM. Structure 22: 1687-1692.

## Claims

1. A VHH antibody which specifically recognizes the pre-fusion state of an HSV-1 gB polypeptide.

2. The VHH antibody of claim 1 which further specifically recognises the pre-fusion state of an HSV-2 gB polypeptide.

3. The VHH antibody of claim 1 or 2 that neutralizes HSV-1 and HSV-2.

4. The VHH antibody of any one of claims 1-3 which
(a) specifically recognizes an epitope of an HSV-1 gB polypeptide in the pre-fusion state comprising or consisting of amino acids A106, N108, G121, A122, D285, E286, G292, F294, Y296, H311, T312, S313, Y314, A315, D317, P348, K349, N585, Y640, E651, A654, Y655, S656, H657, Q658, L659, and D663 as shown in SEQ ID NO: 1 wherein D285 may be replaced by N285 and/or S312 may be replaced by T313, and/or
(b) specifically recognizes an epitope of an HSV-2 gB polypeptide in the pre-fusion state comprising or consisting of amino acids V101, G116, A117, D280, E281, G287, F289, Y291, H306, T307, S308, Y309, A310, R313, P343, N582, Y637, E648, A651, Y652, S653, H654, Q655, and D660 as shown in SEQ ID NO: 2 2 wherein S308 may be replaced by T308.

5. The VHH antibody of any one of claims 1-4 which competes with the VHH antibody Nb1_gbHSV as shown in SEQ ID NO: 13 for binding to an HSV-1 gB polypeptide in the pre-fusion state and/or for binding to an HSV-2 gB polypeptide in the pre-fusion state.

6. The VHH antibody of any one of claims 1-9 which comprises
(a) a combination of CDR1, CDR2 and CDR3 sequences as in shown in SEQ. ID NO: 14-16 or
(b) a combination of CDR1, CDR2 and CDR3 sequences which has an identity of at least 80%, at least 90% or at least 95% to a combination of CDR1, CDR2 and CDR3 sequences of (a).

7. The VHH antibody of any one of claims 1-6 which comprises
(a) a VHH sequence as shown in SEQ. ID NO: 13 or
(b) a VHH sequence which has an identity of at least 90%, at least 95% or at least 98% to a VHH sequence of (a).

8. The VHH antibody of any one of claims 6-7 which comprises a VHH sequence comprising amino acids Q1, A26, S27, G28, R29, M32, G34, A35, Y39, L49, T52, T54, D55, R56, S58, T59, N60, Y61, K66, R100, W101, R102, G103, M104 of SEQ ID NO: 13.

9. The VHH antibody of any one of the preceding claims which is a fully disulfide-bonded VHH antibody and has a melting temperature of at least 95°C.

10. The VHH antibody of any one of the preceding claims which is covalently or non-covalently conjugated to a heterologous moiety and which particularly is fused to an immunoglobulin Fc fragment.

11. A nucleic acid molecule encoding a VHH antibody according to any one of claims 1-10, preferably in operative linkage with a heterologous expression control sequence, or a vector comprising said nucleic acid molecule.

12. A recombinant cell or non-human organism transformed or transfected with a nucleic acid molecule or a vector according to claim 11, which is particularly probiotic microorganism secreting a VHH antibody according to any one of claims 1-10 and being capable of inhabiting a mammalian, e.g., human mucosa, for example of the female genital tract.

13. A pharmaceutical composition comprising the VHH antibody of any one of claims 1-10, the nucleic acid molecule or the vector of claim 11 or the cell or organism of claim 12 and a pharmaceutically acceptable carrier.

14. The VHH antibody of any one of claims 1-10, the nucleic acid molecule or the vector of claim 11, the cell or organism claim 12 or the pharmaceutical composition of claim 13 for use in medicine, particularly in the prevention, treatment and/or mitigation of a condition caused by, associated with and/or accompanied by an infection with HSV-1 or HSV-2.

15. The VHH antibody of any one of claims 1-10, the nucleic acid molecule or the vector of claim 11, the cell or organism claim 12 or the pharmaceutical composition of claim 13 for the use of claim 14 as a combination with at least one further therapy, and wherein the further therapy particularly comprises administration of a herpesvirus DNA polymerase inhibitor.
